(19) Europäisches Patentamt / European Patent Office / Office européen des brevets

(11) EP 2 520 663 A1

(12) EUROPEAN PATENT APPLICATION

(43) Date of publication:
**07.11.2012 Bulletin 2012/45**

(51) Int Cl.:
***C12Q 1/68*** *(2006.01)*

(21) Application number: **12166067.4**

(22) Date of filing: **27.04.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.05.2011 JP 2011102987**
**30.09.2011 JP 2011218114**
**30.03.2012 JP 2012082391**

(71) Applicant: **ARKRAY, Inc.**
**Kyoto-shi, Kyoto 601-8045 (JP)**

(72) Inventors:
- **Hirai, Mitsuharu**
  **Kyoto-shi, Kyoto 602-0008 (JP)**
- **Kurose, Kaoru**
  **Kyoto-shi, Kyoto 602-0008 (JP)**
- **Iguchi, Aki**
  **Kyoto-shi, Kyoto 602-0008 (JP)**

(74) Representative: **Jones, Elizabeth Louise**
**Dehns**
**St Bride's House**
**10 Salisbury Square**
**London**
**EC4Y 8JD (GB)**

(54) **Gene mutation detection probe**

(57) A gene mutation detection probe for detection of a target gene mutation in a base sequence encoding a gene of interest that includes the target gene mutation and a non-target gene mutation, the probe including at least one oligonucleotide selected from the group consisting of oligonucleotides P1, P1-1, P1', and P1'-1. For example, the P1 oligonucleotide is an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and has an identity of at least 80% with a base sequence complementary to a part or all of the base sequence encoding the gene of interest.

FIG. 1A

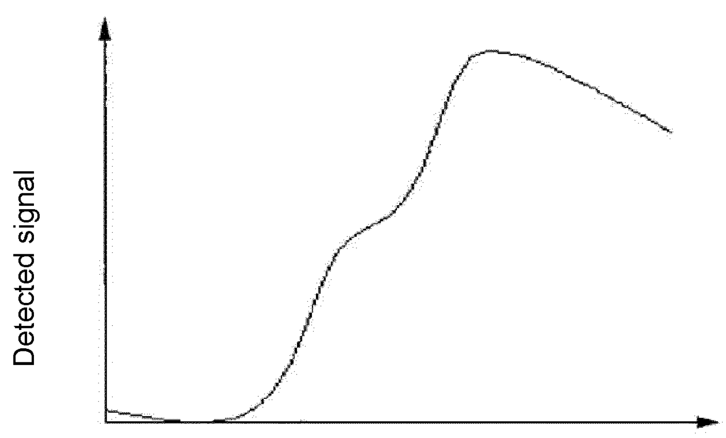


EP 2 520 663 A1

FIG. 1B

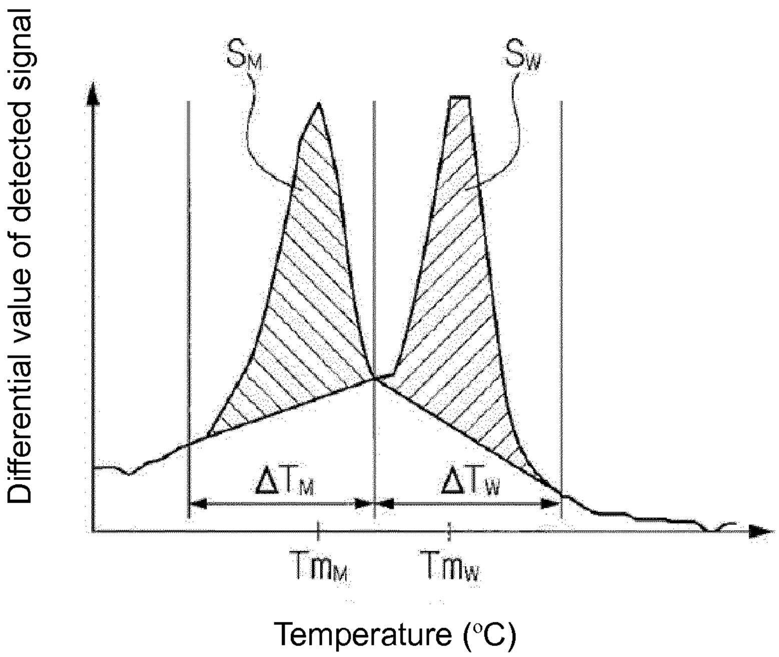
Differential value of detected signal
$S_M$
$S_W$
$\Delta T_M$
$\Delta T_W$
$Tm_M$
$Tm_W$
Temperature (°C)

SEQUENCE LISTING

<110> ARKRAY, INC.

<120> GENE MUTATION DETECTION PROBE

<130> 42.13.112808

<150> JP2011-218114
<151> 2011-09-30

<150> JP2011-102987
<151> 2011-05-02

<150> JP2012-082391
<151> 2012-03-30

<160> 35

<170> PatentIn version 3.4

<210> 1
<211> 701
<212> DNA
<213> Homo sapiens

<220>
<221> misc_feature
<222> (491)..(491)
<223> r is g or a.

<220>
<221> misc_feature
<222> (501)..(501)
<223> r is g or a.

<220>
<221> misc_feature
<222> (507)..(507)
<223> r is g or a.

<400> 1
cagctgaggc acagccaaga gctctggctg tattaatgac ctaagaagtc accagaaagt 60

cagaagggat gacatgcaga ggcccagcaa tctcagctaa gtcaactcca ccagcctttc 120

tagttgccca ctgtgtgtac agcaccctgg tagggaccag agccatgaca gggaataaga 180

ctagactatg cccttgagga gctcacctct gttcagggaa acaggcgtgg aaacacaatg 240

gtggtaaaga ggaaagagga caataggatt gcatgaaggg gatggaaagt gcccagggga 300

ggaaatggtt acatctgtgt gaggagtttg gtgaggaaag actctaagag aaggctctgt 360

ctgtctgggt ttggaaggat gtgtaggagt cttctagggg gcacaggcac actccaggca 420

taggtaaaga tctgtaggtg tggcttgttg ggatgaattt caagtatttt ggaatgagga 480

cagccataga racaagggca rgagagrggc gatttaatag attttatgcc aatggctcca 540

cttgagtttc tgataagaac ccagaaccct tggactcccc agtaacattg attgagttgt 600

ttatgatacc tcatagaata tgaactcaaa ggaggtcagt gagtggtgtg tgtgtgattc 660

tttgccaact tccaaggtgg agaagcctct tccaactgca g 701

<210> 2
<211> 21
<212> DNA
<213> Artificial

<220>
<223> 3PB-CYP3A4\201E1B-R3

<400> 2
taaatcgccg ctctcctgcc c 21

<210> 3
<211> 501
<212> DNA
<213> Artificial

<220>
<223> SEQ3

<220>
<221> misc_feature
<222> (251)..(251)
<223> r is g or a.

<220>
<221> misc_feature
<222> (256)..(256)
<223> s is g or c.

<220>
<221> misc_feature
<222> (260)..(260)
<223> y is t or c.

<220>
<221> misc_feature
<222> (273)..(273)
<223> s is g or c.

<400> 3
gggctcgagg cgtcccccgg ggagtcgcct cttagcggtg cgtccgggct agcggcgagg 60

ggccgcccca agtcttccca ccgccgccac cttagcagcc cgacttgggg cctggaaagt 120

ggagcacgcg gaggtgggag ggccctgcac gcggccccg gtggggaagg ggacgggcca 180

gggattcaga ctcgggctct cccctcagga tgcagcaccg aggcttcctc ctcctcaccc 240

tcctcgccct rctggsgcty acctccgcgg tcsccaaaaa gaaaggtgat gggggatgat 300

cgaaggaggg ctggggacgg gcaggcgagg cccctccact tctgggctgg gccgcctggg 360

ttcctagcct ggaaccccag gaaggcggct cccgagggag tctccccgtg ccccagtcct 420

gaactctgtt cctcgcgcgt tgtagataag gtgaagaagg gcggcccggg gagcgagtgc 480

gctgagtggg cctgggggcc c 501

<210> 4

<211> 17
<212> DNA
<213> Artificial

<220>
<223> SEQ 4

<400> 4
ctccagcagg gcgagga 17

<210> 5
<211> 17
<212> DNA
<213> Artificial

<220>
<223> SEQ 5

<400> 5
caccagcagg gcgagga 17

<210> 6
<211> 18
<212> DNA
<213> Artificial

<220>
<223> SEQ 6

<400> 6
cctcgcccta ctggagct 18

<210> 7
<211> 18
<212> DNA
<213> Artificial

<220>
<223> SEQ 7

<400> 7
cctcgcccta ctggtgct 18

<210> 8
<211> 588
<212> DNA
<213> Homo sapiens

<220>
<221> misc_feature
<222> (322)..(322)
<223> r is g or a.

<220>
<221> misc_feature
<222> (355)..(355)
<223> r is g or a.

<220>
<221> misc_feature

<222> (364)..(364)
<223> r is g or a.

<220>
<221> misc_feature
<222> (370)..(370)
<223> r is g or a.

<400> 8
ctgctcagag ctcacagacc tgggtgcccg ggccctgacg actcacacag gcccggagct 60

gggagaggat atggtgcagg gtgtgaaggg gctggtccaa gacatccccc agggctgggt 120

cagtgtcagc ggtggcctcc agaaccttca gcgtcagggc cagctcagcc tccaaagcca 180

cggggcgctc cctcacctga ggagaggtga gaaagagcag gtgagggggg aggtgagggg 240

aacaggttgg gggaggagga tagagaggaa caagtgaagg tgacaggcac aggggagagg 300

gcacagccag tgtggtcagg trggagcaga gggaaggggt agcaggtgtg gggaraggag 360

agarggacar tggagaagga gaaggtgaag gggccactac agagccaggt gagcagggct 420

gggagggcag gggtgggcct gactccccct ctcacctgca gctgcctcag gtcccaggtc 480

ctggggaaga ggcgggagcg gcacttgcag tccttcagca gaagcgactc ttcctagaca 540

gcaaaggcac aggttagccc cagcaggagg ggtggaggtt agaccact 588

<210> 9
<211> 18
<212> DNA
<213> Artificial

<220>
<223> SEQ 9

<400> 9
tccatctctc ctctcccc 18

<210> 10
<211> 18
<212> DNA
<213> Artificial

<220>
<223> SEQ 10

<400> 10
tccgtctctc ctctcccc 18

<210> 11
<211> 18
<212> DNA
<213> Artificial

<220>
<223> SEQ 11

<400> 11
gggagaggag agatggac 18

<210> 12
<211> 18
<212> DNA
<213> Artificial

<220>
<223> SEQ 12

<400> 12
gggagaggag agacggac 18

<210> 13
<211> 401
<212> DNA
<213> Homo sapiens

<220>
<221> misc_feature
<222> (187)..(187)
<223> w is a or t.

<220>
<221> misc_feature
<222> (201)..(201)
<223> m is a or c.

<400> 13
aactatagtt gatacctata aaattgactt cacaatgcac tattgggtca aaatttccag 60

tttgaaaaac agtgtgccta gacagtctgg tgaagttcag tttccaaact aatcatccgc 120

aggtctccta gttcctcaaa tgcacatctt ctgactcctt taggggtttc ctatcctcta 180

gtgttgwtgt cttcctgggg magccctcta ttctgctctt gattgaattt cctctccgat 240

atcacctgct tgcctggcta tgatgatcat tgctgctgta gtgattcata aatgtatatt 300

cctaactgtg gttgcgttcc tgaattctgt atgatgtttc cagcagcatt ccaaacagtt 360

tcatgtagcc ttctgttgta cccttcatct tgaactcagc a 401

<210> 14
<211> 22
<212> DNA
<213> Artificial

<220>
<223> SEQ 14

<400> 14
gctgccccag gaagacacca ac 22

<210> 15
<211> 22
<212> DNA
<213> Artificial

<220>
<223> SEQ 15

<400> 15
gctgccccag gaagacagca ac 22

<210> 16
<211> 24
<212> DNA
<213> Artificial

<220>
<223> SEQ 16

<400> 16
ttgctgtctt cctggggcag cccc 24

<210> 17
<211> 24
<212> DNA
<213> Artificial

<220>
<223> SEQ 17

<400> 17
ttggtgtctt cctggggcag cccc 24

<210> 18
<211> 21
<212> DNA
<213> Artificial

<220>
<223> SEQ 18

<400> 18
ctaccccagg aagacatcaa c 21

<210> 19
<211> 21
<212> DNA
<213> Artificial

<220>
<223> SEQ 19

<400> 19
tgatgtcttc ctggggggagc c 21

<210> 20
<211> 501
<212> DNA
<213> Homo sapiens

<220>
<221> misc_feature
<222> (247)..(247)
<223> y is c or t.

<220>
<221> misc_feature

<222> (251)..(251)
<223> k is g or t.

<400> 20
gtttttatg ccatgtatat ttctctatgt gtagcctttg cctaaaacaa cacatgatta 60

atatttgttc attgttcctt ttgctatcac ccctgtctag gatctacaca ttaagaaaca 120

aagacatgaa cgtctccatg gaaagactgg gaaaatggat tgcaggttct agcaggatgt 180

cataataaat ggtgcatatc cagagtgcaa gatgattcag tctcaccaag aacactgaaa 240

gtcacayggc kaccagcatt attgtgataa gaactactat tttgggagat agtttagcaa 300

aggtgccatg tagaaattga ttaagtcaga ggtatcttta acttgccacc acagagaaga 360

gattaatttc atatacttcc attgagaaga gagataagaa tacaaaacca agctgatttg 420

caggagtaaa cttgatattc aaatactatt tcctgaatga cattttctga gacatgctaa 480

ttgtaattac tttcagcttc a 501

<210> 21
<211> 18
<212> DNA
<213> Artificial

<220>
<223> SEQ 21

<400> 21
ggttgccatg tgactttc 18

<210> 22
<211> 18
<212> DNA
<213> Artificial

<220>
<223> SEQ 22

<400> 22
ggtggccatg tgactttc 18

<210> 23
<211> 16
<212> DNA
<213> Artificial

<220>
<223> SEQ 23

<400> 23
aagtcacatg gcaacc 16

<210> 24
<211> 16
<212> DNA
<213> Artificial

<220>

<223> SEQ 24

<400> 24
aagtcacatg gccacc 16

<210> 25
<211> 40
<212> DNA
<213> Artificial

<220>
<223> SEQ 25

<400> 25
agccatagag acaagggcaa gagagaggcg atttaataga 40

<210> 26
<211> 40
<212> DNA
<213> Artificial

<220>
<223> SEQ 26

<400> 26
agccatagag acaagggcag gagagaggcg atttaataga 40

<210> 27
<211> 40
<212> DNA
<213> Artificial

<220>
<223> SEQ 27

<400> 27
agccatagag acaagggcaa gagaggggcg atttaataga 40

<210> 28
<211> 40
<212> DNA
<213> Artificial

<220>
<223> SEQ 28

<400> 28
agccatagag acaagggcag gagaggggcg atttaataga 40

<210> 29
<211> 480
<212> DNA
<213> Homo sapiens

<220>
<221> misc_feature
<222> (227)..(227)
<223> y is t or c.

```
<220>
<221>  misc_feature
<222>  (234)..(234)
<223>  r is g or a.

<220>
<221>  misc_feature
<222>  (234)..(234)
<223>  s is g or c.

<220>
<221>  misc_feature
<222>  (245)..(245)
<223>  y is t or c.

<400>  29
agtagaattt ggattcaaag tagccatgag atatatagca tgtgttggag ggaaaaaaac     60

cccacaacat atatattctc ttataggtta ttagacccac aacatatatg tcctcttata    120

ggttattaga tgtcttagct gcaaggaaag atccaagtgg attatctgga gatgttctga    180

taaatggagc accgcgacct gccaatttca aatgtaattc aggttaygtg gtasaagtaa    240

gtatyagtgg gtttgcattt tctgtttcct ctgtttctat atgggtaagt gctttsgctg    300

atagttcaat gtgcttccag ttgattatgt gacatggtcc tagaactgac gttctttaca    360

gcagcttttc ttaatttctc atagacactt atgtgaaaag gcagggagaa tctggaatat    420

ggcccttgta aggacagtga taccaattct agtttttgta tcatttctaa aatgatacat    480


<210>  30
<211>  24
<212>  DNA
<213>  Artificial

<220>
<223>  5FL-ABCG2 Q126X-T-R1

<400>  30
cccacttata cttacttata ccac                                            24


<210>  31
<211>  50
<212>  DNA
<213>  Artificial

<220>
<223>  ABCG2-Q126X-50-F-WT

<400>  31
caaatgtaat tcaggttacg tggtacaagt aagtattagt gggtttgcat                50


<210>  32
<211>  50
<212>  DNA
<213>  Artificial

<220>
<223>  ABCG2-Q126X-50-F-mt
```

```
<400>  32
caaatgtaat tcaggttacg tggtataagt aagtattagt gggtttgcat              50


<210>  33
<211>  40
<212>  DNA
<213>  Artificial

<220>
<223>  ABCG2-Q126X-40-F-WTC

<400>  33
ggttacgtgg tacaagtaag tatcagtggg tttgcatttt                         40


<210>  34
<211>  40
<212>  DNA
<213>  Artificial

<220>
<223>  ABCG2-Q126X-40-F-mtC

<400>  34
ggttacgtgg tataagtaag tatcagtggg tttgcatttt                         40


<210>  35
<211>  21
<212>  DNA
<213>  Artificial

<220>
<223>  3PB-CYP3A4 1B-R3TK

<400>  35
taaatcgcct ctctcctgcc c                                             21
```

## Description

## BACKGROUND

Technical Field

The present invention relates to a gene mutation detection probe.

Related Art.

The term "gene mutation" means that a base in a base sequence encoding a gene is different from that found in the majority of a certain population, and refers to, for example, a difference in gene structure (base sequence) such as single base substitution, deletion, insertion, or gene fusion.

The term "genetic polymorphism" refers to one embodiment of the term "gene mutation", and refers to a state in which the frequency of a mutation or allele (allelic gene) observed at the highest frequency in a certain population is 99% or lower — in other words, a state in which the total frequency of less frequent mutations or alleles is 1% or higher. Although the frequency of "1%" is arbitrarily set, the frequency of "1%" actually has significance. Specifically, since most new mutations that occur in each generation are removed from the population by natural selection, the equilibrium frequency thereof can be theoretically calculated. The frequency of 1%, which is higher than the equilibrium frequency, is employed as a criterion for the "polymorphism." That is, the occurrence of a polymorphism indicates that the frequency of the mutation in the population has been increased through a certain mechanism, and the mutation has been retained over many generations.

It has been pointed out that a gene mutation is associated with the morbidity rate of various diseases or with the frequency of occurrence of side effects caused by drug administration. Therefore, it is thought that prediction of the frequency of development of various diseases, drug resistance or the like will be enabled by detection of genetic polymorphisms. Accordingly, a method is desired whereby a gene mutation can be measured quickly and correctly in a simple manner at low cost.

Types of genetic polymorphism and gene mutation include: single base substitution, which is substitution of a single base in a base sequence; deletion mutation, which refers to deletion of one or more bases; insertion mutation, which refers to insertion of one or more bases; and gene fusion. The genetic polymorphism and the gene mutation are hereinafter collectively referred to as "gene mutation."

At present, a method whereby a mutation-containing region is amplified by a PCR method, a nucleic acid probe labeled with a fluorescent dye is thereafter hybridized to the target nucleic acid thereof, the amount of reduction of the emission from the fluorescent dye is measured, and the mutation in the base sequence is analyzed based on the results of the melting curve analysis, is known as a method of measuring a gene mutation, (see Japanese Patent Application Laid-Open (JP-A) No. 2002-119291).

## SUMMARY OF THE INVENTION

However, in order to detect a gene mutation by using the above-described method in which a nucleic acid probe is used, it is necessary to use nucleic acid probes having sequences suitable for respective mutations. In addition, when a nucleic acid probe is used to carry out melting curve analysis, the presence of a mutation, other than the gene mutation to be detected, within a nucleic acid probe causes variations of the melting temperature (Tm value) due to an influence from the mutation other than the gene mutation to be detected, thereby making it difficult to specifically detect the gene mutation to be detected.

In consideration of the current circumstances as described above, it has been desired to further develop a technique for accurate detection of a particular gene mutation to be detected, which is not affected by a gene mutation other than the gene mutation to be detected even when a gene-encoding base sequence includes multiple gene mutations.

An object of the present invention is to provide a gene mutation detection probe that enables specific detection of only a particular gene mutation to be detected from multiple gene mutations contained in a gene-encoding base sequence without being affected by the types of bases at non-target gene mutations, and a method of detecting a gene mutation using the same. Another object of the present invention is to provide a reagent kit for detection of a gene mutation in which the gene mutation detection probe is used.

Aspects of the present invention include the following:

<1> A gene mutation detection probe for detection of a target gene mutation in a base sequence encoding a gene of interest that includes the target gene mutation and a non-target gene mutation, the probe including at least one oligonucleotide selected from the group consisting of oligonucleotides P1, P1-1, P1', and P1'-1:

the P1 oligonucleotide being an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and has an identity of at least 80% with a base sequence complementary to a part or all of the base sequence encoding the gene of interest;
the P1-1 oligonucleotide being an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and hybridizes with an oligonucleotide having the same base sequence as the base sequence encoding the gene of interest under stringent conditions;
the P1' oligonucleotide being an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and has an identity of at least 80% with a part or all of the base sequence encoding the gene of interest; and
the P1'-1 oligonucleotide being an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and hybridizes with an oligonucleotide having a base sequence complementary to the base sequence encoding the gene of interest under stringent conditions.

<2> The gene mutation detection probe of <1>, which satisfies any one of the following conditions (a) to (f):

(a) when the wild-type base at the base position of the non-target gene mutation is one of A or T, and the mutant base of the non-target gene mutation is the other one of A or T, the base complementary to neither the wild-type base nor the mutant base of the non-target gene mutation or the base different from both the wild-type base and the mutant base of the non-target gene mutation is C or G;
(b) when the wild-type base at the base position of the non-target gene mutation is one of C or G, and the mutant base of the non-target gene mutation is the other one of C or G, the base complementary to neither the wild-type base nor the mutant base of the non-target gene mutation or the base different from both the wild-type base and the mutant base of the non-target gene mutation is A or T;
(c) when the wild-type base at the base position of the non-target gene mutation is one of A or C, and the mutant base of the non-target gene mutation is the other one of A or C, the base complementary to neither the wild-type base nor the mutant base of the non-target gene mutation is A or C, and the base different from both the wild-type base and the mutant base of the non-target gene mutation is T or G;
(d) when the wild-type base at the base position of the non-target gene mutation is one of A or G, and the mutant base of the non-target gene mutation is the other one of A or G, the base complementary to neither the wild-type base nor the mutant base of the non-target gene mutation is A or G, and the base different from both the wild-type base and the mutant base of the non-target gene mutation is T or C;
(e) when the wild-type base at the base position of the non-target gene mutation is one of T or C, and the mutant base of the non-target gene mutation is the other one of T or C, the base complementary to neither the wild-type base nor the mutant base of the non-target gene mutation is T or C, and the base different from both the wild-type base and the mutant base of the non-target gene mutation is A or G; or
(f) when the wild-type base at the base position of the non-target gene mutation is one of T or G, and the mutant base of the non-target gene mutation is the other one of T or G, the base complementary to neither the wild-type base nor the mutant base of the non-target gene mutation is T or G, and the base different from both the wild-type base and the mutant base of the non-target gene mutation is A or C.

<3> The gene mutation detection probe of <1> or <2>, wherein the oligonucleotide is a fluorescently-labeled oligonucleotide.
<4> The gene mutation detection probe of <3>, wherein the base at the 3' or 5' end of the fluorescently-labeled oligonucleotide is a cytosine labeled with a fluorescent dye.
<5> The gene mutation detection probe of <3> or <4>, wherein the fluorescently-labeled oligonucleotide is fluorescently-labeled at any of the first to the third bases from the 3' or 5' end.
<6> The gene mutation detection probe of any one of <1> to <5>, which is a probe for melting curve analysis.
<7> The gene mutation detection probe of any one of <3> to <6>, wherein the fluorescence intensity when the fluorescently-labeled oligonucleotide is hybridized with a target sequence thereof is decreased or increased as compared to the fluorescence intensity when the fluorescently-labeled oligonucleotide is not hybridized with the target sequence.
<8> The gene mutation detection probe of any one of <3> to <7>, wherein the fluorescence intensity when the fluorescently-labeled oligonucleotide is hybridized with a target sequence thereof is decreased as compared to the fluorescence intensity when the fluorescently-labeled oligonucleotide is not hybridized with the target sequence.
<9> A method of detecting a gene mutation including detecting a target gene mutation to be detected using the gene mutation detection probe of any one of <1> to <8>. Alternatively expressed, use of the probe of any one of <1> to <8> for detecting a gene mutation including detecting a target gene mutation.
<10> A method of detecting a target gene mutation including:

(I) contacting the gene mutation detection probe of any one of <3> to <8> with a single-stranded nucleic acid in said sample, to obtain a hybrid;
(II) dissociating the hybrid by changing the temperature of the sample containing the hybrid, and measuring the change in fluorescence signal due to the dissociation of the hybrid;
(III) obtaining the Tm value, which is the dissociation temperature of the hybrid, based on the change in fluorescence signal; and
(IV) detecting the presence of the target gene mutation on the single-stranded nucleic acid in the sample based on the Tm value.

<11> The method of detecting a target gene mutation of <10>, further including amplifying the nucleic acid before or simultaneously with obtaining the hybrid in (I).
<12> A gene mutation detection reagent kit that includes the gene mutation detection probe of any one of <1> to <8>.
<13> The gene mutation detection reagent kit of <12>, further including primers capable of amplifying a base sequence having a region to which the gene mutation detection probe of any one of <1> to <8> hybridizes. The invention also extends to the use of said kit for detecting a target gene mutation.
<14> The method of detecting a gene mutation of <9>, wherein the method includes:

(I) contacting the gene mutation detection probe of any one of <3> to <8> with a single-stranded nucleic acid in a sample, to obtain a hybrid;
(II) dissociating the hybrid by changing the temperature of the sample containing the hybrid, and measuring the change in fluorescence signal due to the dissociation of the hybrid;
(III) obtaining the Tm value, which is the dissociation temperature of the hybrid, based on the change in fluorescence signal; and
(IV) detecting the presence of the target gene mutation on the single-stranded nucleic acid in the sample based on the Tm value.

According to the present invention, a gene mutation detection probe that enables specific detection of a particular gene mutation to be detected without being affected by a gene mutation other than the gene mutation to be detected, even when a gene-encoding base sequence includes multiple gene mutations, and a method of detecting a gene mutation using the same can be provided. According to the present invention, a gene mutation detection reagent kit, in which the gene mutation detection probe is employed, can also be provided.

## BRIEF DESCRIPTION OF THE DRAWINGS

Fig. 1A is an example of a melting curve of a nucleic acid mixture, and Fig. 1B is an example of a differential melting curve of a nucleic acid mixture thereof.
Figs. 2A to 2P are differential melting curves of the samples according to Example 1 according to the present invention.
Figs. 3A to 3H are differential melting curves of the samples according to Example 2 according to the present invention.
Figs. 4A to 4P are differential melting curves of the samples according to Comparative Example 1.

## DETAILED DESCRIPTION OF THE INVENTION

The gene mutation detection probe according to the present invention is a gene mutation detection probe for detection of a target gene mutation in a base sequence encoding a gene of interest that includes the target gene mutation and a non-target gene mutation, the probe including at least one oligonucleotide selected from the group consisting of oligonucleotides P1, P1-1, P1', and P1'-1.
Due to this configuration, the gene mutation detection probe according to the present invention specifically detects a particular gene mutation to be detected without being affected by a gene mutation other than the gene mutation to be detected, even when a gene-encoding base sequence includes multiple gene mutations.

The method of detecting a gene mutation according to the present invention includes detecting a target gene mutation to be detected using at least one of the gene mutation detection probe for detection of a gene mutation; therefore, the detection can be carried out without being affected by a gene mutation other than the gene mutation to be detected even when a gene-encoding base sequence includes multiple gene mutations.

The gene mutation detection reagent kit according to the present invention includes the gene mutation detection probe for detection of a gene mutation.

In the present invention, the descriptions of the base sequences of the sample nucleic acid in a sample to be detected and the gene mutation detection probe or primer shall also apply to complementary base sequences thereof, respectively, unless otherwise specified. Further, when the description of a particular base sequence is applied to a complementary base sequence thereof, descriptions of base sequences recognized by the particular base sequence in the present invention should be applied provided that the recognition by the particular base sequence should be replaced with recognition by a complementary base sequence of the particular base sequence, within a range of the common general technical knowledge of those skilled in the art.

In the present invention, the term "Tm value" is defined as the temperature at which a double-stranded nucleic acid dissociates (dissociation temperature: Tm), and is generally defined as the temperature at which the absorbance at 260 nm has increased by 50% of the total increase in absorbance resulting from complete dissociation of the double-stranded nucleic acid. More specifically, when a solution containing a double-stranded nucleic acid such as a double-stranded DNA is heated, the absorbance at 260 nm of the double-stranded nucleic acid gradually increases. This is because the hydrogen bonds between both strands of the double-stranded DNA are broken by heating, thereby dissociating the double-stranded DNA into single-stranded DNAs (melting of DNA). When the double-stranded DNA has completely dissociated into single-stranded DNAs, the single-stranded DNAs exhibit an absorbance that is about 1.5 times the absorbance at the time of the initiation of the heating (i.e., the absorbance when the entire DNA is in the form of double-stranded DNA), which serves as an indicator of the completion of the melting. The Tm value is defined based on this phenomenon.

In the present invention, when the phrase "the first to third bases from the 3' end" is used in connection to an oligonucleotide sequence, it is assumed that the base at the 3' end of the oligonucleotide chain is the first base from the 3' end. Similarly, when the phrase "the first to third bases from the 5' end" is used in connection to an oligonucleotide sequence, it is assumed that the base at the 5' end of the oligonucleotide chain is the first base from the 5' end.

In the present specification, the scope of the term "process" includes not only a discrete process, but also a process that cannot be clearly distinguished from another process as long as the expected effect of the process of interest is achieved.

In the present specification, any numerical range expressed using "to" refers to a range including the numerical values before and after "to" as the minimum and maximum values, respectively.

In a case in which the amount of a component that may be included in the composition is indicated in the present invention, when there are multiple substances corresponding to the component in the composition, the indicated amount means the total amount of the multiple substances present in the composition, unless specifically stated otherwise.

The present invention is described below.

<Gene Mutation Detection Probe>

The gene mutation detection probe according to the present invention is a gene mutation detection probe for detection of a target gene mutation in a base sequence encoding a gene of interest that includes the target gene mutation and a non-target gene mutation, the probe including at least one oligonucleotide selected from the group consisting of oligonucleotides P1, P1-1, P1', and P1'-1.

Due to this configuration, the gene mutation detection probe according to the present invention specifically detects a particular gene mutation to be detected without being affected by a gene mutation other than the gene mutation to be detected, even when a gene-encoding base sequence includes multiple gene mutations.

The P1 oligonucleotide is an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and has an identity of at least 80% with a base sequence complementary to a part or all of the base sequence encoding the gene of interest.

The P1-1 oligonucleotide is an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and hybridizes with an oligonucleotide having the same base sequence as the base sequence encoding the gene of interest under stringent conditions.

The P1' oligonucleotide is an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and has an identity of at least 80% with a part or all of the base sequence encoding the gene of interest.

The P1'-1 oligonucleotide is an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and hybridizes with an oligonucleotide having a base sequence complementary to the base sequence encoding the gene of interest under stringent conditions.

The "base position corresponding to the target gene mutation" in the present invention refers to the base position in an oligonucleotide that corresponds to the base position of the target gene mutation in a sample base sequence containing the target gene mutation and a non-target gene mutation when the base sequence of the oligonucleotide and the sample base sequence are so aligned as to provide the highest match therebetween. Similarly, the "base position corresponding to the non-target gene mutation" in the present invention refers to the base position in an oligonucleotide that corresponds to the base position of the non-target gene mutation in a sample base sequence containing the target gene mutation and the non-target gene mutation when the base sequence of the oligonucleotide and the sample base sequence are so aligned as to provide the highest match therebetween. The wild-type base of the target gene mutation refers to the wild-type base at the base position of the target gene mutation (i.e., at the base position at which mutation to a mutant base of the target gene mutation may occur).

Adenine (A) and thymine (T) form hydrogen bonds therebetween, and guanine (G) and cytosine (C) form hydrogen bonds therebetween. The AT base pair forms two hydrogen bonds whereas the GC base pair forms three hydrogen bonds. Thus, hydrogen bonds are formed between A and T, and between G and C, thereby maintaining the double helix structure. Other combinations of bases cannot form sufficient hydrogen bonds for maintaining the double helix structure.

For example, when the combination of the wild-type base and the mutant base at the base position of the non-target gene mutation contained in a sample nucleic acid is a combination of A and T, and the base complementary to neither the wild-type base nor the mutant base of the non-target gene mutation in the gene mutation detection probe is C or G, the mutant base of the non-target gene mutation and the wild-type base at the base position of the non-target gene mutation (a base selected from A or T) does not form hydrogen bonds with the base (C or G) complementary to neither the wild-type base nor the mutant base of the non-target gene mutation, and, therefore, the Tm value of the gene mutation detection probe is not affected by the non-target gene mutation.

In contrast, if the base complementary to neither the wild-type base nor the mutant base of the non-target gene mutation in the gene mutation detection probe were replaced by A, this base would not form hydrogen bonds with the base at the base position of the non-target gene mutation in the sample nucleic acid when the base at the base position of the non-target gene mutation were A, but forms hydrogen bonds when the base at the base position of the non-target gene mutation were T. In this circumstance, the Tm value of the gene mutation detection probe would be affected not only by the target gene mutation, but also by the non-target gene mutation, as a result of which it would be impossible to accurately detect the state of the target gene mutation using such a gene mutation detection probe outside the scope of the present invention.

In the case in which the target gene mutation in the base sequence encoding a gene of interest and containing multiple gene mutations is detected using a gene mutation detection probe having homology with a base sequence complementary to the base sequence encoding the gene of interest, influence from a non-target gene mutation can be avoided by setting the base on the probe at the base position corresponding to the non-target gene mutation to be a base (non-complementary base) that binds to neither the normal-type base nor the mutant-type base at the base position of the non-target gene mutation, as described above. Therefore, a gene mutation detection probe composed of the P1 or P1-1 oligonucleotide according to the present invention is capable of specifically detecting the target gene mutation without being affected by the type of base at the base position of the non-target gene mutation.

Similarly, in the case in which the target gene mutation in the base sequence encoding a gene of interest and containing multiple gene mutations is detected using a gene mutation detection probe having homology with the base sequence encoding the gene of interest, influence from a non-target gene mutation can be avoided by setting the base on the probe at the base position corresponding to the non-target gene mutation to be a base (different base) that is different from both the normal-type base and the mutant-type base at the base position of the non-target gene mutation. Therefore, a gene mutation detection probe composed of the P1' or P1'-1 oligonucleotide according to the present invention is capable of specifically detecting the target gene mutation without being affected by the type of base at the base position of the non-target gene mutation.

With the gene mutation detection probe according to the present invention, any mutation selected from single base substitution, deletion, and insertion can be recognized. In particular, the gene mutation detection probe according to the present invention is useful especially as a gene mutation detection probe for recognition of a single base substitution-type mutation (single base mutation), from the view point of the detection sensitivity.

The P1 or P1-1 oligonucleotide according to the present invention has homology with a base sequence that is complementary to the gene-encoding base sequence containing the target gene mutation except at the base position corresponding to the non-target gene mutation.

In contrast, the P1' or P1'-1 oligonucleotide has homology with a base sequence that is the same as the gene-encoding base sequence containing the target gene mutation except at the base position corresponding to the non-target gene mutation.

Any known gene can be applied as the gene of interest in the present invention. The gene of interest in the present invention contains either a structural gene or a transcriptional regulatory region.

The transcriptional regulatory region in the present specification refers to a sequence that frequently occurs in the 5' upstream region of a gene signal sequence (but may alternatively occur in the first intron), and that regulates the transcription of RNA from the gene by an RNA polymerase.

In the case of a structural gene, a gene mutation may be present in an exon region or an intron region. In a more specific embodiment, a gene mutation may be present in an exon region.

The expression "having homology" or "identity" in the present invention may refer to homology or identity of from 80% to 100% between the base sequence of the gene mutation detection probe according to the present invention and the base sequence complementary to a part or all of the base sequence that encodes a gene of interest. From the viewpoint of detection sensitivity, the base sequence of the gene mutation detection probe according to the present invention may have an identity of 85% or higher, an identity of 90% or higher, an identity of 95% or higher, an identity of 96% or higher, an identity of 97% or higher, an identity of 98% or higher, or an identity of 99% or higher.

Homology of 80% or higher provides excellent sensitivity of detection from a sample nucleic acid that contains a gene of interest containing a target gene mutation to be detected.

A "part" of the base sequence that encodes a gene of interest may be, for example, 1-5, 1-10, 1-20, 1-30, 1-40, 1-50, 1-100, 1-250, 1-500, or 1-1000 or more mer (nucleotides) in length and contains the target gene mutation and the non-target gene mutation. The "part" of the base sequence that encodes a gene of interest is preferably the same length as the probe of the present invention as described herein and preferably is the part to which said probe corresponds, i.e. to which part the probe or its complementary sequence may bind.

In an embodiment, the P1 or P1-1 oligonucleotide according to the present invention is complementary (completely complementary) to a part of the base sequence encoding the gene of interest that contains the target gene mutation and the non-target gene mutation, except at the base position corresponding to the non-target gene mutation and, optionally, the base position corresponding to the target gene mutation. In another embodiment, the P1 or P1-1 oligonucleotide according to the present invention is completely complementary to the entire base sequence encoding the gene of interest that contains the target gene mutation and the non-target gene mutation, except at the base position corresponding to the non-target gene mutation and, optionally, the base position corresponding to the target gene mutation.

In an embodiment, the P1' or P1'-1 oligonucleotide according to the present invention is identical with a part of the base sequence encoding the gene of interest that contains the target gene mutation and the non-target gene mutation, except at the base position corresponding to the non-target gene mutation and, optionally, the base position corresponding to the target gene mutation. In another embodiment, the P1' or P1'-1 oligonucleotide according to the present invention is identical with the entire base sequence encoding the gene of interest that contains the target gene mutation and the non-target gene mutation, except at the base position corresponding to the non-target gene mutation and, optionally, the base position corresponding to the target gene mutation.

Hybridization as referred to herein is performed under stringent conditions. The hybridization under stringent conditions in the present invention is described below.

The hybridization may be carried out according to a known method or a method corresponding thereto, such as a method described in Molecular Cloning 3rd (J. Sambrook et al., Cold Spring Harbor Lab. Press, 2001). This document is incorporated herein by reference.

The term "stringent conditions" means conditions in which specific hybridization occurs to form a hybrid whereas non-specific hybridization does not occur. Typical stringent conditions are, for example, conditions in which hybridization is carried out at a potassium concentration of from about 25 mM to about 50 mM and a magnesium concentration of from about 1.0 mM to about 5.0 mM. One example of such conditions in the present invention is conditions in which hybridization is carried out in Tris-HCl (pH 8.6) at 25 mM KCl and 1.5 mM $MgCl_2$; however, hybridization conditions in the present invention are not limited thereto. Another example of stringent conditions is described in Molecular Cloning 3rd (J. Sambrook et al., Cold Spring Harbor Lab. Press, 2001). This document is incorporated herein by reference. Those skilled in the art can readily choose such conditions by changing the hybridization reaction, the salt concentration of the hybridization reaction solution, and the like.

The P1, P1-1, P1', and P1'-1 oligonucleotides according to the present invention are described below. Although the description is given referring to the base sequence represented by SEQ ID NO:1 or SEQ ID NO:2 as an example, as necessary, the P1, P1-1, P1', and P1'- oligonucleotides according to the present invention are not limited thereto.

The base sequence represented by SEQ ID NO:1 is a base sequence encoding a mutant CYP3A4 gene (CYP3A4*1B rs2740574), which is a drug-metabolizing enzyme. The CYP3A4 gene, which encodes CYP3A4, is located on human chromosome 7. It has been reported that a CYP3A4 gene mutation is relevant to drug metabolism, for example, of an immunosuppressant tacrolimus (Clin. Pharmacol. Ther., 2006, Vol. 80, pp. 179-191). Therefore, the result of detection of a mutation in the CYP3A4 gene is quite important information in the selection of a more effective disease treatment method.

The rs2740574, which is a mutant CYP3A4 gene, has a mutant base at the 501st base of SEQ ID NO:1. The rs number is a registration number in the dbSNP database of the National Center for Biotechnology Information (//www.ncbi.nlm.nih.gov/projects/SNP/) (the same shall apply hereinafter). In the wild type CYP3A4 gene, the base corresponding to the 501 st base of the base sequence represented by SEQ ID NO:1 is A (adenine), whereas that in the mutant gene is G (guanine).

The base sequence represented by SEQ ID NO:2 is one example of the gene mutation detection probe according to the present invention for detection of a target gene mutation at the 501st base in the base sequence represented by SEQ ID NO:1.

The base sequence encoding a gene of interest in the present invention contains multiple gene mutations. More specifically, the base sequence encoding the gene of interest contains two or more gene mutations, and may contain, for example, three gene mutations or four gene mutations.

The target gene mutation may be appropriately selected, as necessary, from among the multiple gene mutations contained in the base sequence encoding the gene of interest. For example, the gene mutation at the 501st position of the base sequence represented by SEQ ID NO:1, which encodes the CYP3A4 gene, may be selected as the target gene mutation to be detected. Therefore, in the case of the base sequence represented by SEQ ID NO:1 as an example, the mutant base of the target gene mutation according to the present invention is A (adenine) or G (guanine), and the wild-type base at the base position of the target gene mutation according to the present invention is the other one of A (adenine) or G (guanine).

Referring to the base sequence represented by SEQ ID NO:1 as an example, the base at the base position corresponding to the target gene mutation that is complementary to either the wild-type base or the mutant base of the target gene mutation in the P1 or P1-1 oligonucleotide is T (thymine) or C (cytosine).

Referring to the base sequence represented by SEQ ID NO:1 as an example, a mutant base of a non-target gene mutation in the explanation of the P1 or P1-1 oligonucleotide is R at the 507th position of the base sequence represented by SEQ ID NO:1. At the 507th base position, A (adenine) is present in the wild type, whereas G (guanine) is present in the mutant gene.

Referring to the base sequence represented by SEQ ID NO:1 as an example, the base at the base position corresponding to the non-target gene mutation that is complementary to neither the wild-type base nor a mutant base of the non-target gene mutation in the P1 or P1-1 oligonucleotide is A (adenine) or G (guanine).

Referring to the base sequence represented by SEQ ID NO:1 as an example, the base sequence that is complementary to a base sequence formed by the 496th to the 516th bases of the base sequence encoding the CYP3A4 gene corresponds to the base sequence complementary to the base sequence encoding the gene of interest in the explanation of the P1 oligonucleotide.

Referring to the base sequence represented by SEQ ID NO:1 as an example, an oligonucleotide having the same base sequence as a base sequence formed by the 496th to the 516th bases of the base sequence encoding the CYP3A4 gene corresponds to the oligonucleotide having the same base sequence as the base sequence encoding the gene of interest in the explanation of the P1-1 oligonucleotide .

Referring to the base sequence represented by SEQ ID NO:1 as an example, the same base as either the wild-type base or a mutant base of the target gene mutation in the P1' or P1'-1 oligonucleotide is A (adenine) or G (guanine).

Referring to the base sequence represented by SEQ ID NO:1 as an example, the base different from both the wild-type base and a mutant base of the non-target gene mutation in the P1' or P1'-1 oligonucleotide is T (thymine) or C (cytosine).

In regard to other elements in the explanation of the P1' or P1'-1 oligonucleotide that are common to the P1 or P1-1 oligonucleotide, all of the descriptions contained in the explanation of the P1 or P1-1 oligonucleotide shall apply.

The target gene mutation in the present invention encompasses all of the case in which the base at the base position of the target gene mutation varies between one wild-type base and one mutant base, the case in which the base at the base position of the target gene mutation varies between one wild-type base and two mutant bases, and the case in which the base at the base position of the target gene mutation varies between one wild-type base and three mutant bases.

Examples of the P1, P1-1, P1', and P1'-1 oligonucleotides in the present invention include those which satisfy any one of the following conditions (a) to (f):

(a) when the wild-type base at the base position of the non-target gene mutation is one of A or T, and the mutant base of the non-target gene mutation is the other one of A or T, the base complementary to neither the wild-type base nor the mutant base of the non-target gene mutation or the base different from both the wild-type base and the mutant base of the non-target gene mutation is C or G;

(b) when the wild-type base at the base position of the non-target gene mutation is one of C or G, and the mutant base of the non-target gene mutation is the other one of C or G, the base complementary to neither the wild-type base nor the mutant base of the non-target gene mutation or the base different from both the wild-type base and the mutant base of the non-target gene mutation is A or T;

(c) when the wild-type base at the base position of the non-target gene mutation is one of A or C, and the mutant base of the non-target gene mutation is the other one of A or C, the base complementary to neither the wild-type base nor the mutant base of the non-target gene mutation is A or C, and the base different from both the wild-type base and the mutant base of the non-target gene mutation is T or G;

(d) when the wild-type base at the base position of the non-target gene mutation is one of A or G, and the mutant base of the non-target gene mutation is the other one of A or G, the base complementary to neither the wild-type base nor the mutant base of the non-target gene mutation is A or G, and the base different from both the wild-type base and the mutant base of the non-target gene mutation is T or C;

(e) when the wild-type base at the base position of the non-target gene mutation is one of T or C, and the mutant base of the non-target gene mutation is the other one of T or C, the base complementary to neither the wild-type base nor the mutant base of the non-target gene mutation is T or C, and the base different from both the wild-type base and the mutant base of the non-target gene mutation is A or G; or

(f) when the wild-type base at the base position of the non-target gene mutation is one of T or G, and the mutant base of the non-target gene mutation is the other one of T or G, the base complementary to neither the wild-type base nor the mutant base of the non-target gene mutation is T or G, and the base different from both the wild-type base and the mutant base of the non-target gene mutation is A or C.

The oligonucleotide according to the present invention encompasses an oligonucleotide obtained by modifying the P1, P1-1, P1', or P1'-1 oligonucleotide by insertion, deletion, or substitution of at least one base.
An oligonucleotide obtained by modifying the P1, P1-1, P1', or P1'-1 oligonucleotide by insertion, deletion, or substitution of at least one base may be employed if the oligonucleotide exerts an effect comparable to that exerted by the P1, P1-1, P1', or P1'-1 oligonucleotide. In the case of insertion, deletion, or substitution of at least one base, the position of each insertion, deletion or substitution is not particularly limited. The number of inserted, deleted, or substituted bases may be, for example, one base, or two or more bases, and may vary depending on the entire length of the oligonucleotide. The number of inserted, deleted, or substituted bases may be, for example, from one to ten bases, or from one to five bases.

The length of the P1, P1-1, P1', or P1'-1 oligonucleotide according to the present invention may be, for example, from 11 mer to 60 mer. An oligonucleotide length within this range provides excellent sensitivity of detection of a gene mutation.
The P1, P1-1, P1', or P1'-1 oligonucleotide according to the present invention may have a length of from 12 mer to 55 mer, a length of from 15 mer to 45 mer, or a length of from 18 mer to 40 mer. For example, the length may be in a range of from 12 mer to 55 mer. An oligonucleotide length in this range tends to provide, for example, a higher detection sensitivity.
Further, the adjustment of the base length of the P1, P1-1, P1', or P1'-1 oligonucleotide allows, for example, adjustment of the Tm value, which is the dissociation temperature of the hybrid formed by the P1, P1-1, P1', or P1'-1 oligonucleotide and a complementary strand thereof (target sequence), to a desired value

A method for designing the gene mutation detection probe according to the present invention is described below by way of specific examples. However, the present invention is not limited thereto.

The base sequence represented by SEQ ID NO:3 is a sequence containing rs20542. The base sequence represented by SEQ ID NO:3 includes a target gene mutation at the 25 1 st base position, and, in the vicinity thereof, a non-target gene mutation at the 256th base position.
In this case, in the gene mutation detection probe corresponding to the P1 or P1-1 oligonucleotide, a base complementary to either the wild-type base or the mutant base at the 251st base is selected as the base at the corresponding base position in the P1 or P1-1 oligonucleotide, and a base complementary to neither the wild-type base nor the mutant base of the non-target gene mutation at the 256th base is selected as the base at the corresponding base position in the P1 or P1-1 oligonucleotide.
Specific examples thereof include the gene mutation detection probes represented by SEQ ID NO:4 or SEQ ID NO:5 in Table 1.
In the gene mutation detection probe corresponding to the P1' or P1'-1 oligonucleotide, the same base as either the wild-type base or the mutant base at the 25 1 st base is selected as the base at the corresponding base position in the P1' or P1'-1 oligonucleotide, and a base different from both the wild-type base and the mutant base of the non-target gene mutation at the 256th base is selected as the base at the corresponding base position in the P1' or P1'-1 oligonucleotide.
Specific examples thereof include the gene mutation detection probes represented by SEQ ID NO:6 or SEQ ID NO:7 in Table 1.

In the case in which any of the gene mutation detection probes represented by SEQ ID NOs:4 to 7 is used to detect the presence or absence of the target gene mutation at the 25 1 st position in the base sequence represented by SEQ ID NO:3, the presence or absence of the target gene mutation at the 251 st position can be specifically detected even when a non-target gene mutation occurs in the vicinity of the 251 st base.
In Table 1, the underlined capital letters represent the target gene mutation to be detected; and the capital letters without underlining represent the non-target gene mutation. The same shall apply hereinafter.

Table 1

| | |
|---|---|
| SEQ ID NO:4 | (Fluorescent Dye)-cTccagCagggcgagga |
| SEQ ID NO:5 | (Fluorescent Dye)-cAccagCagggcgagga |
| SEQ ID NO:6 | (Fluorescent Dye)-cctcgccctActggAgct |
| SEQ ID NO:7 | (Fluorescent Dye)-cctcgccctActggTgct |

The base sequence represented by SEQ ID NO:8 is a sequence containing rs11881222. The base sequence represented by SEQ ID NO:8 includes a target gene mutation at the 355th position and, in the vicinity thereof, a non-target gene mutation at the 364th position.
In this case, in the gene mutation detection probe corresponding to the P1 or P1-1 oligonucleotide, a base complementary to either the wild-type base or the mutant base at the 355th base is selected as the base at the corresponding base position in the P1 or P1-1 oligonucleotide, and a base complementary to neither the wild-type base nor the mutant base of the non-target gene mutation at the 364th base is selected as the base at the corresponding base position in the P1 or P1-1 oligonucleotide. Specific examples thereof include the gene mutation detection probes represented by SEQ ID NO:9 or SEQ ID NO:10 in Table 2.
In the gene mutation detection probe corresponding to the P1' or P1'-1 oligonucleotide, the same base as either the wild-type base or the mutant base at the 355th base is selected as the base at the corresponding base position in the P1' or P1'-1 oligonucleotide, and a base different from both the wild-type base and the mutant base of the non-target gene mutation at the 364th base is selected as the base at the corresponding base position in the P1' or P1'-1 oligonucleotide. Specific examples thereof include the gene mutation detection probes represented by SEQ ID NO:11 or SEQ ID NO:12 in Table 2.

In the case in which any of the gene mutation detection probes represented by SEQ ID NOs:9 to 12 is used to detect the presence or absence of the target gene mutation at the 355th position in the base sequence represented by SEQ ID NO:8, the presence or absence of the target gene mutation at the 355th position can be specifically detected even when a non-target gene mutation occurs in the vicinity of the 355th base.

Table 2

| | |
|---|---|
| SEQ ID NO:9 | tccAtctctcctCtcccc-(Fluorescent Dye) |
| SEQ ID NO:10 | tccGtctctcctCtcccc-(Fluorescent Dye) |
| SEQ ID NO:11 | gggaGaggagagaTggac-(Fluorescent Dye) |
| SEQ ID NO:12 | gggaGaggagagaCggac-(Fluorescent Dye) |

The base sequence represented by SEQ ID NO:13 is a sequence containing rs80230660. The base sequence represented by SEQ ID NO:13 includes a target gene mutation at the 201st position and, in the vicinity thereof, a non-target gene mutation at the 187th position.
In this case, in the gene mutation detection probe corresponding to the P1 or P1-1 oligonucleotide, a base complementary to either the wild-type base or the mutant base at the 201st base is selected as the base at the corresponding base position in the P1 or P1-1 oligonucleotide, and a base complementary to neither the wild-type base nor the mutant base of the non-target gene mutation at the 187th base is selected as the base at the corresponding base position in the P1 or P1-1 oligonucleotide. Specific examples thereof include the gene mutation detection probes represented by SEQ ID NO:14 or SEQ ID NO:15 in Table 3.
In the gene mutation detection probe corresponding to the P1' or P1'-1 oligonucleotide, the same base as either the wild-type base or the mutant base at the 20 1 st base is selected as the base at the corresponding base position in the P1' or P1'-1 oligonucleotide, and a base different from both the wild-type base and the mutant base of the non-target gene mutation at the 187th base is selected as the base at the corresponding base position in the P1' or P1'-1 oligonucleotide. Specific examples thereof include the gene mutation detection probes represented by SEQ ID NO:16 or SEQ ID NO:17 in Table 3.

In the case in which any of the gene mutation detection probes represented by SEQ ID NOs:14 to 17 is used to detect the presence or absence of the target gene mutation at the 20 1 st position in the base sequence represented by SEQ ID NO:13, the presence or absence of the target gene mutation at the 201 st position can be specifically detected even when a non-target gene mutation occurs in the vicinity of the 20 1 st base.

Table 3

| | |
|---|---|
| SEQ ID NO:14 | gctGccccaggaagacaCcaac-(Fluorescent Dye) |
| SEQ ID NO:15 | gctGccccaggaagacaGcaac-(Fluorescent Dye) |
| SEQ ID NO:16 | ttgCtgtcttcctggggCagcccc-(Fluorescent Dye) |
| SEQ ID NO:17 | ttgGtgtcttcctggggCagcccc-(Fluorescent Dye) |

The base sequence represented by SEQ ID NO:13 includes a target gene mutation at the 187th position and, in the vicinity thereof, a non-target gene mutation at the 201 st position.
In this case, in the gene mutation detection probe corresponding to the P1 or P1-1 oligonucleotide, a base complementary to either the wild-type base nor the mutant base at the 187th base is selected as the base at the corresponding base position in the P1 or P1-1 oligonucleotide, and a base complementary to neither the wild-type base nor the mutant base of the non-target gene mutation at the 20 1 st base is selected as the base at the corresponding base position in the P1 or P1-1 oligonucleotide. Specific examples thereof include the gene mutation detection probes represented by SEQ ID NO:18 in Table 4.
In the gene mutation detection probe corresponding to the P1' or P1'-1 oligonucleotide, the same base as either the wild-type base or the mutant base at the 187th base is selected as the base at the corresponding base position in the P1' or P1'-1 oligonucleotide, and a base different from both the wild-type base and the mutant base of the non-target gene mutation at the 20 1 st base is selected as the base at the corresponding base position in the P1' or P1'-1 oligonucleotide. Specific examples thereof include the gene mutation detection probes represented by SEQ ID NO:19 in Table 4.

In the case in which the gene mutation detection probes represented by SEQ ID NO:18 or 19 is used to detect the presence or absence of the target gene mutation at the 187th position in the base sequence represented by SEQ ID NO:13, the presence or absence of the target gene mutation at the 187th position can be specifically detected even when a non-target gene mutation occurs in the vicinity of the 187th base.

Table 4

| | |
|---|---|
| SEQ ID NO:18 | ctAccccaggaagacaTcaac-(Fluorescent Dye) |
| SEQ ID NO:19 | tgAtgtcttcctggggGagcc-(Fluorescent Dye) |

The base sequence represented by SEQ ID NO:20 is a sequence containing rs74182491. The base sequence represented by SEQ ID NO:20 includes a target gene mutation at the 247th position and, in the vicinity thereof, a non-target gene mutation at the 251 st position.
In this case, in the gene mutation detection probe corresponding to the P1 or P1-1 oligonucleotide, a base complementary to either the wild-type base or the mutant base at the 247th base is selected as the base at the corresponding base position in the P1 or P1-1 oligonucleotide, and a base complementary to neither the wild-type base nor the mutant base of the non-target gene mutation at the 25 1 st base is selected as the base at the corresponding base position in the P1 or P1-1 oligonucleotide. Specific examples thereof include the gene mutation detection probes represented by SEQ ID NO:21 or SEQ ID NO:22 in Table 5.
In the gene mutation detection probe corresponding to the P1' or P1'-1 oligonucleotide, the same base as either the wild-type base or the mutant base at the 247th base is selected as the base at the corresponding base position in the P1' or P1'-1 oligonucleotide, and a base different from both the wild-type base and the mutant base of the non-target gene mutation at the 251 st base is selected as the base at the corresponding base position in the P1' or P1'-1 oligonucleotide. Specific examples thereof include the gene mutation detection probes represented by SEQ ID NO:23 or SEQ ID NO:24 in Table 5.

In the case in which any of the gene mutation detection probes represented by SEQ ID NOs:21 to 24 is used to detect the presence or absence of the target gene mutation at the 247th position in the base sequence represented by SEQ ID NO:20 the presence or absence of the target gene mutation at the 247th position can be specifically detected even when a non-target gene mutation occurs in the vicinity of the 247th base.

Table 5

| | |
|---|---|
| SEQ ID NO:21 | ggtTgccAtgtgactttc-(Fluorescent Dye) |
| SEQ ID NO:22 | ggtGgccAtgtgactttc-(Fluorescent Dye) |
| SEQ ID NO:23 | aagtcacaTggcAacc-(Fluorescent Dye) |
| SEQ ID NO:24 | aagtcacaTggcCacc-(Fluorescent Dye) |

According to the present invention, a gene mutation detection probe with high detection sensitivity can be provided not only in the case in which the non-target gene mutation present in the vicinity of the target gene mutation is a single base substitution mutation as described above, but also in a case in which a deletion mutation or an insertion mutation is present.

When the non-target gene mutation is a deletion mutation, a gene mutation detection probe corresponding to the P1 or P1-1 oligonucleotide according to the present invention can be obtained by the above-described method, except that a base that is not complementary to the deleted base is selected as the base at the corresponding base position in the P1 or P1-1 oligonucleotide (i.e., the base in the P1 or P1-1 located at the base position corresponding to the deleted base). More specifically, in the case in which A is the deleted base, the base at the corresponding base position in the P1 or P1-1 oligonucleotide (i.e., the base in the P1 or P1-1 located at the base position corresponding to the deleted base A) may be at least one base selected from A, C and G, which are bases non-complementary to A.

Similarly, when the non-target gene mutation is a deletion mutation, a gene mutation detection probe corresponding to the P1' or P1-1' oligonucleotide according to the present invention can be obtained by the above-described method, except that a base that is different from the deleted base is selected as the base at the corresponding base position in the P1' or P1'-1 oligonucleotide (i.e., the base in the P1' or P1'-1 located at the base position corresponding to the deleted base). More specifically, in the case in which A is the deleted base, the base at the corresponding base position in the P1' or P1'-1 oligonucleotide (i.e., the base in the P1' or P1'-1 located at the base position corresponding to the deleted base A) may be at least one base selected from G, T and C, which are bases different from A.

When the non-target gene mutation is an insertion mutation, a gene mutation detection probe corresponding to the P1 or P1-1 oligonucleotide according to the present invention can be obtained by the above-described method, except that a base that is not complementary to the inserted base is selected as the base at the corresponding base position in the P1 or P1-1 oligonucleotide (i.e., the base in the P1 or P1-1 located at the base position corresponding to the inserted base). More specifically, in the case in which G is the inserted base, the base at the corresponding base position in the P1 or P1-1 oligonucleotide (i.e., the base in the P1 or P1-1 located at the base position corresponding to the inserted base G) may be at least one base selected from A, T and G, which are bases non-complementary to G.

Similarly, when the non-target gene mutation is an insertion mutation, a gene mutation detection probe corresponding to the P1' or P1-1' oligonucleotide according to the present invention can be obtained by the above-described method, except that a base that is different from the inserted base is selected as the base at the corresponding base position in the P1' or P1'-1 oligonucleotide (i.e., the base in the P1' or P1'-1 located at the base position corresponding to the inserted base). More specifically, in the case in which G is the inserted base, the base at the corresponding base position in the P1' or P1'-1 oligonucleotide (i.e., the base in the P1' or P1'-1 located at the base position corresponding to the inserted base A) may be at least one base selected from A, T and C, which are bases different from G.

The P1, P1-1, P1', or P1'-1 oligonucleotide according to the present invention may be a fluorescently-labeled oligonucleotide. The fluorescently-labeled oligonucleotide may be a fluorescently-labeled oligonucleotide such that the fluorescence intensity when the oligonucleotide is hybridized with a target sequence thereof is decreased (quenched) or increased as compared to the fluorescence intensity when the oligonucleotide is not hybridized with the target sequence thereof. In particular, the fluorescently-labeled oligonucleotide may be a fluorescently-labeled oligonucleotide such that the fluorescence intensity when the oligonucleotide is hybridized with the target sequence thereof is decreased as compared to the fluorescence intensity when the oligonucleotide is not hybridized with the target sequence thereof. A probe that utilizes a "fluorescence quenching phenomenon" as described above is generally referred to as guanine quenching probe, and known as Q PROBE®. In particular, the fluorescently-labeled oligonucleotide may be an oligonucleotide designed to have cytosine (C) at its 3' or 5' end, and labeled with a fluorescent dye such that fluorescence emission thereof is reduced when the terminal C approaches guanine (G).

A known detection method other than the detection method using a Q PROBE® may also be applied. Examples of such a detection method include a TAQ-MAN probe method, a a hybridization probe method, a molecular beacon method, and a MGB probe method.

The fluorescent dye is not particularly limited, and examples of the fluorescent dye include fluorescein, phosphor, rhodamine and polymethine dye derivatives. Examples of commercially available products of such fluorescent dyes include Pacific Blue, BODIPY FL, FluorePrime, Fluoredite, FAM, Cy3 and Cy5, and TAMRA.

The detection conditions of the fluorescently-labeled oligonucleotide are not particularly limited, and may be decided, as appropriate, in accordance with the fluorescent dye to be used. For example, Pacific Blue can be detected at a detection wavelength of from 445 nm to 480 nm, TAMRA can be detected at a detection wavelength of from 585 nm to 700 nm, and BODIPY FL can be detected at a detection wavelength of from 520 nm to 555 nm.

By using a probe having such a fluorescent dye, hybridization and dissociation of the probe can be readily confirmed based on the change in fluorescence signal thereof. Attachment of a fluorescent dye to the oligonucleotide may be carried out according to an ordinary method, such as a method described in JP-A No. 2002-119291.

In addition, the fluorescently-labeled oligonucleotide may have, for example, a phosphate group added to its 3' end. Addition of a phosphate group to the 3' end of the fluorescently-labeled oligonucleotide suppresses elongation of the probe itself by a gene amplification reaction. As described below, a DNA for which the presence or absence of a mutation should be detected (target DNA) may be prepared using a gene amplification method such as PCR. When the fluorescently-labeled oligonucleotide that has a phosphate group added to its 3' end is used, the amplification reaction can be carried out even in the presence of the oligonucleotide in a reaction solution of the amplification reaction.

A similar effect can be obtained also by adding a labeling substance (a fluorescent dye) as described above to the 3' end.

The P1, P1-1, P1', or P1'-1 oligonucleotide may be used as a gene mutation detection probe for specifically detecting a particular gene mutation to be detected without being affected by a gene mutation other than the gene mutation to be detected, even when a gene-encoding base sequence contains multiple gene mutations.

In addition, the gene mutation detection probe may be used as a probe for melting curve analysis.

<Primers>

In the below-described gene mutation detection method according to the present invention, primers are used in the case in which the sequence containing a target gene mutation to be detected is amplified by a PCR method.

The primers that may be used in the present invention are not particularly limited as long as the primers are capable of amplifying, for example, a nucleic acid that contains bases corresponding to the 501st base and the 507th base of the base sequence represented by SEQ ID NO:1, which are gene mutation sites in a gene of interest to be detected.

The primers to be applied to the PCR method are not particularly limited as long as the primers are capable of amplifying a region to which the gene mutation detection probe according to the present invention can hybridize. Those skilled in the art are able to design such primers, as appropriate, based on, for example, the base sequence represented by SEQ ID NO:1. In general, the length and the Tm value of each primer may be a length of from 12 mer to 40 mer and a Tm of from 40°C to 70°C, or a length of from 16 mer to 30 mer and a Tm of from 55°C to 60°C.

The length of each primer in the primer set does not have to be the same as each other, and the Tm values of both primers of the primer set may be substantially the same (or the difference between the Tm values of both primers may be within 5°C).

The method of detecting a gene mutation is not particularly limited as long as the fluorescently-labeled nucleotide described above is used as a probe in the method. A method of detecting a gene mutation using Tm analysis is described below as an example of the method of detecting a gene mutation in which the P1, P1-1, P1', or P1'-1 oligonucleotide described above is used as a probe.

<Gene Mutation Detection Method>

The method of detecting a gene mutation according to the present invention is a gene mutation detection method which includes detecting a gene mutation using at least one type of gene mutation detection probe including the fluorescently-labeled P1, P1-1, P1', or P1'-1 oligonucleotide described above (hereinafter sometimes referred to as "fluorescently-labeled gene mutation detection probe").

Since at least one of the fluorescently-labeled gene mutation detection probes described above is employed in the method of detecting a gene mutation according to the present invention, a gene mutation can be detected easily with high sensitivity.

In addition, the method of detecting a gene mutation according to the present invention may be employed as a method of detecting a gene mutation in various human genes, and may include the below-described processes (I) to (IV), and may include the below-described process (V). The method of detecting a gene mutation according to the present invention has the feature of using the above-described gene mutation detection probe, and other configurations, conditions and the like are not particularly limited by the description below.

Process (I): contacting the fluorescently-labeled gene mutation detection probe with a single-stranded nucleic acid in a sample, to obtain a hybrid.
Process (II): dissociating the hybrid by changing the temperature of the sample containing the hybrid, and measuring the change in fluorescence signal due to the dissociation of the hybrid.
Process (III): measuring the Tm value, which is the dissociation temperature of the hybrid, based on the change in fluorescence signal.
Process (IV): detecting the presence of the target gene mutation on the single-stranded nucleic acid in the sample, based on the Tm value.
Process (V): determining the abundance ratio of single-stranded nucleic acid having the gene mutation in the total single-stranded nucleic acids contained in the sample, based on the presence of the gene mutation.

Furthermore, the method according to the present invention may further include amplifying the nucleic acid before the obtaining the hybrid in the process (I) or simultaneously with obtaining the hybrid in the process (I), in addition to the processes (I) to (IV) or in addition to the processes (I) to (V).
The measurement of the Tm value in the process (III) may include not only measuring the dissociation temperature of the hybrid, but also measuring the differential values of the fluorescence signal that changes according to the temperature when the hybrid is melted.
In the present invention, the nucleic acid in the sample may be single-stranded nucleic acid or double-stranded nucleic acid. In the case in which the nucleic acid is double-stranded nucleic acid, the method may include, for example, melting (dissociating) the double-stranded nucleic acid in the sample into single-stranded nucleic acids by heating before being hybridized with the fluorescently-labeled gene mutation detection probe. The dissociation of a double-stranded nucleic acid into single-stranded nucleic acids enables hybridization with the fluorescently-labeled gene mutation detection probe.
In the present invention, the nucleic acid contained in the sample to be detected may be, for example, a nucleic acid originally contained in a biological sample, or an amplification product obtained by amplifying a region of the gene of interest that contains a mutated site by PCR or the like using a nucleic acid originally contained in a biological sample as a template with a view to improving the detection accuracy. The length of the amplification product is not particularly limited, and may be, for example, a length of from 50 mer to 1000 mer, or a length of from 80 mer to 200 mer. Furthermore, the nucleic acid in the sample may be, for example, a cDNAthat has been synthesized from RNAs derived from a biological sample (e.g., total RNAs, mRNAs, etc.) by RT-PCR (Reverse Transcription PCR).
In the present invention, the addition ratio (molar ratio) of the gene mutation detection probe according to the present invention relative to the nucleic acids in the sample is not particularly limited. The amount of the gene mutation detection probe to be added may be, for example, no more than 1 times (by mol) the amount of DNAs in the sample. From the viewpoint of ensuring a sufficient detection signal, the addition ratio of the gene mutation detection probe according to the present invention to be added relative to the nucleic acids in the sample (in a molar ratio) may be 0.1 or lower.
The "nucleic acids in the sample" may be, for example, the total of nucleic acids to be detected that have the gene mutation to be detected and nucleic acids, other than the nucleic acids to be detected, that do not have the gene mutation, or the total of amplification products containing a detection target sequence having the gene mutation to be detected and amplification products containing a sequence, other than the detection target sequence, that does not have the gene mutation. Although the ratio of the nucleic acid to be detected relative to nucleic acids in the sample is usually unknown in advance, the consequential addition ratio of the gene mutation detection probe relative to the nucleic acids to be detected (or the amplification products containing a sequence to be detected) (in a molar ratio) may be 10 or lower. The addition ratio of the gene mutation detection probe relative to the nucleic acids to be detected (or the amplification products containing a sequence to be detected) (in a molar ratio) may be 5 or lower, or 3 or lower. The lower limit of the ratio is not particularly limited, and may be, for example, 0.001 or higher, 0.01 or higher, or 0.1 or higher.
The above-described addition ratio of the fluorescently-labeled gene mutation detection probe according to the present invention relative to DNAs may be, for example, a molar ratio relative to double-stranded nucleic acids or a molar ratio relative to single-stranded nucleic acids.
In the present invention, the measurement of the change in the signal caused by a temperature change for determining the Tm value may be carried out by measuring the absorbance at 260 nm on the basis of the principle described above. However, the measurement may be carried out by measuring a signal which is based on a signal from the label attached to the fluorescently-labeled gene mutation detection probe, and which varies in accordance with the degree of the formation of a hybrid of a single-stranded DNA and the gene mutation detection probe. Therefore, the above-described fluorescently-labeled oligonucleotide may be used as the fluorescently-labeled gene mutation detection probe. Examples of the fluorescently-labeled P1, P1-1, P1', or P1'-1 oligonucleotide (hereinafter sometimes collectively referred to as "fluorescently-labeled oligonucleotide") include a fluorescently-labeled oligonucleotide of which the fluorescence intensity when the oligonucleotide is hybridized with a target sequence thereof is decreased (quenched) as compared to the fluorescence intensity when the oligonucleotide is not hybridized with the target sequence thereof, and a ftuorescently-labeled oligonucleotide of which the fluorescence intensity when the oligonucleotide is hybridized with a target sequence thereof is increased as compared to the fluorescence intensity when the oligonucleotide is not hybridized with the target sequence thereof.

The former fluorescently-labeled oligonucleotide does not show a fluorescence signal or only a weak fluorescence signal when the fluorescently-labeled oligonucleotide forms a hybrid (a double-stranded DNA) with the sequence to be detected; however, the fluorescently-labeled oligonucleotide begins to show a fluorescence signal or shows an increased fluorescence signal when the fluorescently-labeled oligonucleotide is dissociated by heating.

The latter fluorescently-labeled oligonucleotide shows a fluorescence signal when the fluorescently-labeled oligonucleotide forms a hybrid (a double-stranded DNA) with the sequence to be detected; however, the ftuorescently-labeled oligonucleotide shows a decreased fluorescence signal or ceases to show a fluorescent signal when the fluorescently-labeled oligonucleotide is dissociated by heating. Therefore, similar to the measurement of the absorbance at 260 nm described above, the progress of melting can be monitored, and the Tm value can be determined by detecting the change in the fluorescence signal from the fluorescent label under the conditions specific to the fluorescent label (for example, the fluorescence wavelength thereof).

The method for detecting the change in the signal based on the signal from the fluorescent dye in the gene mutation detection method according to the present invention is described below by way of specific examples. The gene mutation detection method according to the present invention has the feature of using the fluorescently-labeled gene mutation detection probe, and other processes and conditions of the method are not limited in any way.

The sample containing a nucleic acid that serves as a template for nucleic acid amplification is not particularly limited as long as the sample contains a nucleic acid, particularly the gene of interest. Examples of such a sample include a sample that is derived from or can be derived from any biological source, examples of which include: a tissue such as colon or lung; a hemocyte such as a leukocyte cell; whole blood; plasma; sputum; a suspension of oral mucosa; a somatic cell of nail, hair or the like; a germ cell; milk; ascitic fluid; a paraffin-embedded tissue; gastric juice; gastric lavage fluid; urine; peritoneal fluid; amniotic fluid; and a cell culture. The method for sampling the sample, the method for preparing the sample containing a nucleic acid, and the like are not limited, and, conventional methods known in the art may be employed therefor. A nucleic acid obtained from such a biological source may be directly used as the template, or may be used after the sample has been subjected to pretreatment that modifies the properties of the sample.

For example, in the case in which whole blood is used as the sample, the isolation of genomic DNA from the whole blood may be carried out by a conventional method known in the art. For example, a commercially available genomic DNA isolation kit (trade name: GFX GENOMIC BLOOD DNA PURIFICATION KIT, available from GE Healthcare Biosciences), etc. may be used.

Next, a fluorescently-labeled gene mutation detection probe including the fluorescently-labeled oligonucleotide is added to the sample containing the isolated genomic DNA.

The fluorescently-labeled gene mutation detection probe may be added to a liquid sample containing the isolated genomic DNA, or may be mixed with the genomic DNA in an appropriate solvent. The solvent is not particularly limited, and examples of the solvent include conventional solvents known in the art, such as: a buffer solution such as Tris-HCl; a solvent containing at least one of KCl, $MgCl_2$, $MgSO_4$, or glycerol; and a PCR reaction solution.

The timing of adding the fluorescently-labeled gene mutation detection probe is not particularly limited. For example, in the case in which an amplification process such as PCR described below is carried out, the fluorescently-labeled gene mutation detection probe may be added to the PCR amplification products after the amplification process is carried out, or may be added before the amplification process is carried out.

In the case in which the ftuorescently-labeled gene mutation detection probe is added before an amplification process such as PCR is carried out, for example, a fluorescent dye or a phosphate group may have been added to the 3' end of the probe, as described above.

The method of amplifying the nucleic acid may be, for example, a method in which a polymerase is employed. Examples thereof include a PCR method, an ICAN method, a LAMP method, and an NASBA method. In the case in which the amplification is carried out by a method in which a polymerase is employed, the amplification may be carried out in the presence of the fluorescently-labeled gene mutation detection probe according to the present invention. Those skilled in the art would be able to easily adjust the reaction conditions of the amplification and the like in accordance with the fluorescently-labeled gene mutation detection probe and the polymerase to be used. In the case in which the amplification is carried out in the presence of the fluorescently-labeled gene mutation detection probe according to the present invention, a gene mutation can be detected by only analyzing the Tm value of the fluorescently-labeled gene mutation detection probe after the amplification of the nucleic acid is carried out, and, therefore, it is not necessary to separate the amplification product after completion of the reaction. Thus, contamination by the amplification product does not occur. In addition, since the detection can be carried out by the same apparatus as the apparatus required for the amplification, conveyance of a vessel is unnecessary, and automatization of the process is facilitated.

The DNA polymerase to be used in the PCR method may be selected, without particular limitation, from DNA polymerases that are usually used for PCR. Examples of the DNA polymerase include GENE TAQ (trade name, manufactured by NIPPON GENE CO., LTD.), PRIMESTAR MAX DNA POLYMERASE (trade name, manufactured by Takara Bio Inc.), and a Taq polymerase.

The amount of the polymerase to be used is not particularly limited as long as a usually-applied polymerase concentration is provided. For example, in the case in which a Taq polymerase is used, the concentration of the Taq polymerase may be, for example, a concentration of from 0.01 U to 100 U relative to 50 μl of the reaction solution. In this range, for example, the sensitivity of the detection of a target gene mutation tends to be increased.

The PCR method may be carried out under the conditions appropriately selected from usually-employed conditions.

When the amplification is carried out, the amplification may be monitored using real-time PCR so that the copy number of the DNA (a sequence to be detected) contained in the sample can be measured. In other words, the proportion of probes forming hybrids is increased as the amplification of the DNA (a sequence to be detected) by PCR proceeds, thereby changing the fluorescence intensity. By monitoring the change in the fluorescence intensity, the copy number and/or the abundance ratio of the sequence to be detected (either a normal DNA or a mutant DNA) contained in the sample can be obtained.

In the gene mutation detection method according to the present invention, the fluorescently-labeled oligonucleotide and a single-stranded nucleic acid in the sample are brought into contact with each other, thereby allowing hybridization thereof. The single-stranded nucleic acid in the sample can be prepared by, for example, dissociating the PCR amplification products obtained in the above-described manner.

The heating temperature employed for dissociation of the PCR amplification products (the heating temperature in the dissociation process) is not particularly limited as long as it is a temperature at which the amplification products can be dissociated. For example, the heating temperature may be in the range of from 85˚C to 95˚C. The heating time is not particularly limited, either. The heating time may be, for example, in the range of from 1 second to 10 minutes, or from 1 second to 5 minutes

The hybridization of the dissociated single-stranded DNA and the fluorescently-labeled oligonucleotide may be carried out by, for example, decreasing, after the dissociation process, the temperature from the heating temperature employed in the dissociation process. The temperature condition for the hybridization may be, for example, in the range of from 40˚C to 50˚C.

The volume and concentration of each component in the reaction solution in the hybridization process are not particularly limited. In regard to specific examples thereof, the concentration of DNAs in the reaction solution may be, for example, a concentration of from 0.01 μM to 1 μM, or a concentration of from 0.1 μM to 0.5 μM. The concentration of the fluorescently-labeled oligonucleotide may be, for example, in a range in which the above-described addition ratio relative to DNAs is satisfied, and may be, for example, a concentration of from 0.001 μM to 10 μM, or a concentration of from 0.001 μM to 1 μM.

The resultant hybrid of the single-stranded DNA and the fluorescently-labeled oligonucleotide is gradually heated, and the change in fluorescence signal caused by the temperature increase is measured. For example, in the case of using Q PROBE®, the fluorescence intensity in the state of being hybridized with the single-stranded DNA is decreased (or quenched) as compared to the fluorescence intensity in the dissociated state. Therefore, for example, the hybrid emitting decreased fluorescence or the quenched hybrid may be gradually heated, and an increase in fluorescence intensity caused by the temperature increase may be measured.

The temperature range in which the change in fluorescence intensity is measured is not particularly limited, and the initial temperature may be, for example, a temperature of from room temperature to 85˚C, or a temperature of from 25˚C to 70˚C. The final temperature may be, for example, a temperature of from 40˚C to 105˚C. The temperature increase rate is not particularly limited, either, and may be, for example, in the range of from 0.1˚C/sec to 20˚C/sec, or in the range of from 0.3˚C/sec to 5˚C/sec.

Next, the change in the signal is analyzed to determine the Tm value. More specifically, the Tm value may be determined by calculating a differential value at each temperature (-d(Fluorescence Intensity)/dT) from the fluorescent intensity obtained, and taking the temperature at which the differential value has the lowest value as the Tm value. The Tm value may alternatively be determined as the point at which the increase in fluorescence intensity per unit time ((Increase in Fluorescence Intensity)/t) has the largest value. On the contrary, in the case in which a probe of whose signal intensity is increased by the formation of the hybrid, rather than a quenching probe, is used as the fluorescently-labeled gene mutation detection probe, the signal analysis and the determination of the Tm value may be carried out by measuring a decrease in fluorescence intensity.

In the present invention, a change in fluorescence signal caused by a temperature increase (preferably an increase in fluorescence intensity) may be measured while heating the hybrid as described above. However, instead of this method, measurement of the change in signal may alternatively be carried out, for example, in the course of hybrid formation. In other words, the temperature of the sample, to which the probe has been added, may be decreased, and the change in fluorescence signal caused by the temperature decrease may be measured in the course of hybrid formation.

For example, in the case in which Q PROBE® is used, the fluorescence intensity is high when the probe is added to the sample since the probe is in the dissociated state. However, when the hybrid is formed by temperature decrease, the fluorescence is decreased (or quenched). Therefore, for example, a decrease in fluorescence intensity caused by temperature decrease may be measured while gradually decreasing the temperature of the heated sample. On the other hand, in the case in which a probe whose signal is increased by hybrid formation is used, the fluorescence intensity is low (or quenched) when the probe is added to the sample since the probe is in the dissociated state. However, when the hybrid is formed by temperature decrease, the fluorescence intensity is increased. Therefore, for example, an increase in fluorescence intensity caused by temperature decrease may be measured while gradually decreasing the temperature of the sample.

A method for detecting multiple gene mutations in a single system is not particularly limited. For example, probes respectively capable of detecting the gene mutations may be mixed in advance, and the resultant mixture may be added to the sample. Alternatively, the probes respectively capable of detecting the gene mutations may be sequentially added to the sample containing a single-stranded nucleic acid.

Here, the "system" refers to one independent reaction system formed by a sample containing a hybrid formed by hybridization of the fluorescently-labeled oligonucleotide and the single-stranded nucleic acid.

<Gene Mutation Detection Reagent Kit>

The gene mutation detection reagent kit according to the present invention includes the above-described gene mutation detection probe.

The gene mutation detection reagent kit includes at least one of the above-described gene mutation detection probes, with which a particular gene mutation to be detected can be specifically detected without being affected by a gene mutation other than the gene mutation to be detected even when a gene-encoding base sequence contains multiple gene mutations. Therefore, for example, detection of a gene mutation tends to be carried out more easily when using the gene mutation detection reagent kit according to the present invention..

In the case in which two or more types of oligonucleotide are contained as gene mutation detection probes, the oligonucleotides may be contained in a mixed state, or may be contained separate from each other.

The two or more types of fluorescently-labeled oligonucleotide may be respectively labeled with fluorescent dyes having different emission wavelengths from each other.

By using the probes labeled with respectively different fluorescent dyes, detection of the signal from each fluorescently-labeled oligonucleotide can simultaneously be carried out even in a single reaction system.

In addition, the gene mutation detection reagent kit according to the present invention may further include primers for amplifying a base sequence having a region to which the above-described gene mutation detection probe can hybridize.

With regard to the probe and the primers that may be included in the gene mutation detection reagent kit, the above descriptions thereof may be applied as they are.

Besides the probe and the primers, the gene mutation detection reagent kit according to the present invention may further include reagents required for carrying out the nucleic acid amplification in the detection method according to the present invention. The gene mutation detection probe, the primers and other reagents may be separately contained, or some of them may be contained in a mixture.

The term "separately contained" may refer to a state in which individual reagents are separated from each other such that the non-contact state therebetween is maintained, and does not necessarily require that the individual reagents be contained in separate containers that can be independently handled.

When the gene mutation detection reagent kit includes a primer set for amplifying a base sequence including a base at the gene mutation site (a region to which the gene mutation detection probe can hybridize), detection of the gene mutation with higher sensitivity, for example, can be achieved.

The gene mutation detection reagent kit according to the present invention may further include an instruction manual that provides instructions for the formation of a differential melting curve for a sample containing a nucleic acid to be detected using the gene mutation detection probe, and for the detection of a gene mutation in a gene-encoding base sequence through Tm value analysis based on the differential melting curve, or instructions that describes various reagents that are contained, or may additionally be contained, in the gene mutation detection reagent kit.

EXAMPLES

The present invention will now be described in detail by way of examples. However, the present invention is not limited to these examples in any way.

Example 1

Tm analysis was carried out using a fully-automated SNP analyzer (trade name: I-DENSY (trademark), manufactured by ARKRAY, Inc.) and the test reagents of the formulation shown in Table 6 below.

Table 6

| (Reaction Solution Volume: 50 μl) | |
|---|---|
| 1 × Gene Taq Universal buffer ※<br>Probe: 3PB-CYP3A4*1 B-R3<br>Template (Complementary Strand) | <br>0.2 μ M<br>0.2 μ M |
| ※ manufactured by NIPPON GENE CO., LTD. | |

Details of the probe (SEQ ID NO:2) used in Table 6 above are shown in Table 7 below, and details of the templates (complementary strands) (ID-1 to ID-16) are shown in Table 8 and Table 9 below. The type of fluorescent dye is indicated in the parentheses at the 3' end of the probe.

The Tm value was calculated using a MELTCALC© 99 FREE (http://www.meltcalc.com/) under the set conditions of: Oligoconc. [μM] of 0.2 and Na eq. [mM] of 50.

In Table 7, "Tm (WT)" represents a Tm value obtained when the template was wild-type with respect to the gene mutation to be detected, and "Tm (mt)" represents a Tm value obtained when the template was mutant-type with respect to the gene mutation to be detected. "Δ" represents the difference between the Tm value in the case of the mt (mutant type) and the Tm value in the case of the WT (wild type).

Table 7

| Name | Sequence | mer | Tm(WT) | Tm(mt) | ⊿ | GC(%) |
|---|---|---|---|---|---|---|
| 3PB-CYP3A4*1B-R3 | taaatcgcc**G**ctctc**<u>C</u>**tgccc-(Pacific Blue) | 21 | 52.7 | 60.8 | 8.1 | 61.9 |
| Underlined capital letter: *1B (target SNP) Capital letter without underlining: a mutation (non-target SNP) other than *1B | | | | | | |

Table 8

| | | |
|---|---|---|
| CYP3A4*1 B-AA-40-F | AGCCATAGAGACAAGGGCA**A**GAGAGaGGCGATTTAATAGA | SEQ ID NO:25 |
| CYP3A4*1 B-GA-40-F | AGCCATAGAGACAAGGGCA**G**GAGAGaGGCGATTTAATAGA | SEQ ID NO:26 |
| CYP3A4*1B-AG-40-F | AGCCATAGAGACAAGGGCA**A**GAGAGgGGCGATTTAATAGA | SEQ ID NO:27 |
| CYP3A4*1B-GG-40-F | AGCCATAGAGACAAGGGCA**G**GAGAGgGGCGATTTAATAGA | SEQ ID NO:28 |

Table 9

| | | Target SNP |
|---|---|---|
| ID—1 | CYP3A4*1 B-AA-40-F | A/A |

(continued)

| | | Target SNP |
|---|---|---|
| ID—2 | CYP3A4*1 B-GA-40-F | G/G |
| ID—3 | CYP3A4*1 B-AG-40-F | A/A |
| ID—4 | CYP3A4*1 B-GG-40-F | G/G |
| ID—5 | CYP3A4*1 B-AA-40-F<br>CYP3A4*1B-GA-40-F | G/G |
| ID—6 | CYP3A4*1B-AA-40-F<br>CYP3A4*1B-AG-40-F | A/A |
| ID—7 | CYP3A4*1B-AA-40-F<br>CYP3A4*1B-GG-40-F | A/G |
| ID—8 | CYP3A4*1B-GA-40-F<br>CYP3A4*1B-AA-40-F | A/G |
| ID—9 | CYP3A4*1B-GA-40-F<br>CYP3A4*1B-AG-40-F | A/G |
| ID—10 | CYP3A4*1B-GA-40-F<br>CYP3A4*1B-GG-40-F | G/G |
| ID—11 | CYP3A4*1B-AG-40-F<br>CYP3A4*1B-AA-40-F | A/A |
| ID—12 | CYP3A4*1B-AG-40-F<br>CYP3A4*1B-GA-40-F | A/G |
| ID—13 | CYP3A4*1B-AG-40-F<br>CYP3A4*1B-GG-40-F | A/G |
| ID—14 | CYP3A4*1B-GG-40-F<br>CYP3A4*1B-AA-40-F | A/G |
| ID—15 | CYP3A4*1B-GG-40-F<br>CYP3A4*1B-GA-40-F | G/G |
| ID—16 | CYP3A4*1B-GG-40-F<br>CYP3A4*1B-AG-40-F | A/G |

For the Tm analysis, treatment at 95˚C for 1 second and treatment at 40˚C for 60 seconds was sequentially carried out, and then the temperature was increased from 40˚C to 80˚C at a temperature increase rate of 1˚C per 3 seconds, during which the change in fluorescence intensity was measured over time.

The fluorescent dye PACIFIC BLUE has an excitation wavelength of from 365 nm to 415 nm, and a detection wavelength of from 445 nm to 480 nm. Using the respective wavelengths, a change in the fluorescence intensity derived from the fluorescently-labeled probe was measured for each sample.

As a result of the Tm analysis, Fig. 2A to Fig. 2P, each showing the change in the fluorescence from the probe, were obtained. In the figures, the vertical axis represents the change in the fluorescence intensity per unit time (d(increase in fluorescence intensity)/t), and the horizontal axis represents the temperature (˚C). Fig. 2A shows the change in the fluorescence observed when ID-1 was used as the template; Fig. 2B shows the change in the fluorescence observed when ID-2 was used as the template; Fig. 2C shows the change in the fluorescence observed when ID-3 was used as the template; Fig. 2D shows the change in the fluorescence observed when ID-4 was used as the template; Fig. 2E shows the change in the fluorescence observed when ID-5 was used as the template; Fig. 2F shows the change in the fluorescence observed when ID-6 was used as the template; Fig. 2G shows the change in the fluorescence observed when ID-7 was used as the template; Fig. 2H shows the change in the fluorescence observed when ID-8 was used as the template; Fig. 2I shows the change in the fluorescence observed when ID-9 was used as the template; Fig. 2J shows the change in the fluorescence observed when ID-10 was used as the template; Fig. 2K shows the change in the fluorescence observed when ID-11 was used as the template; Fig. 2L shows the change in the fluorescence observed when ID-12 was used as the template; Fig. 2M shows the change in the fluorescence observed when ID-13 was used as the template; Fig. 2N shows the change in the fluorescence observed when ID-14 was used as the template; Fig. 2O shows the change in the fluorescence observed when ID-15 was used as the template; and Fig. 2P shows the change in the fluorescence observed when ID-16 was used as the template.

From the results shown in Fig. 2A to Fig. 2P, it was clarified that, when the probe represented by SEQ ID NO: 2 is used, the presence or absence of a gene mutation at the 501st base, which is the target of detection, in the base sequence represented by SEQ ID NO:1 can be detected irrespective of whether or not a non-target gene mutation occurs.

Example 2

Tm analysis was carried out using a fully-automated SNP analyzer IS-5310 (trade name: I-DENSY (trademark), manufactured by ARKRAY, Inc.) and the test reagent of the formulation shown in Table 10 below.

Table 10

| Formulation) | |
|---|---|
| (Reaction Solution Volume : 50 μl) | |
| 1 × PCR buffer | |
| dNTP | 0.2mM |
| $MgCl_2$ | 1.5mM |
| Taq Polymerase (manufactured by ARKRAY, Inc.) | 0.0376U |
| Probe:5FL-ABCG2 Q126X-T-R1 | 0.2 μ M |
| Template | 0.2 μ M |

For the Tm analysis, treatment at 95°C for 1 second and treatment at 40°C for 60 seconds was sequentially carried out, and then the temperature was increased from 40°C to 75°C at a temperature increase rate of 1°C per 3 seconds, during which the change in fluorescence intensity was measured over time. The change in the fluorescence intensity derived from the fluorescently-labeled probe was measured using an excitation wavelength of from 420 nm to 485 nm and a measurement wavelength of from 520 nm to 555 nm.

Table 11

| Type | Template 1 | Template 2 |
|---|---|---|
| Wild Type 1 | ABCG2-Q126X-50-F-WT | |
| Wild Type 2 | ABCG2-Q126X-40-F-WTC | |
| Mutant Type 1 | ABCG2-Q126X-50-F-mt | |
| Mutant Type 2 | ABCG2-Q126X-40-F-mtC | |
| Mixed Type 1 | ABCG2-Q126X-50-F-WT | ABCG2-Q126X-50-F-mt |
| Mixed Type 2 | ABCG2-Q126X-40-F-WTC | ABCG2-Q126X-40-F-mtC |
| Mixed Type 3 | ABCG2-Q126X-50-F-WT | ABCG2-Q126X-40-F-mtC |
| Mixed Type 4 | ABCG2-Q126X-40-F-WTC | ABCG2-Q126X-50-F-mt |

Details of the probe and the single-stranded nucleic acids described above are shown below.

Table 12

| Probe | | | |
|---|---|---|---|
| Name | Sequense | (mer) | |
| 5FL-ABCG2 Q126X-T-R1 | (FL)-cccactTatacttacttAtaccac-(P) | 24 | SEQ ID NO:30 |
| Single-Stranded Nucleic Acid | | | |
| Name | Sequense | (mer) | |
| ABCG2-Q126X-50-F-WT | caaatgtaattcaggttacgtggtaCaagtaagtatTagtgggtttgcat | 50 | SEQ ID NO:31 |
| ABCG2-Q126X-50-F-mt | caaatgtaattcaggttacgtggtaTaagtaagtatTagtgggtttgcat | 50 | SEQ ID NO:32 |
| ABCG2-Q126X-40-F-WTC | ggttaCgtggtaCaagtaagtatCagtgggtttgcatttt | 40 | SEQ ID NO:33 |
| ABCG2-Q126X-40-F-mtC | ggttaCgtggtaTaagtaagtatCagtgggtttgcatttt | 40 | SEQ ID NO:34 |

As a result, it was confirmed that a peak of Wild Types 1 and 2 (in which the 234th base of SEQ ID NO:29 is G) and a peak of Mutant Types 1 and 2 (in which the 234th base of SEQ ID NO:29 is C) can both be detected by using the gene mutation detection probe according to the present invention. It was further confirmed that the peaks can be detected by using the gene mutation detection probe according to the present invention, also when Mixed Types 1 to 4, each of which is a 1:1 mixture of a wild-type single-stranded nucleic acid and a mutant-type single-stranded nucleic acid of human genome type, is used.
The Tm value in the case of Wild Type 1 and the Tm value in the case of Wild Type 2 were both 50˚C, and the Tm value of Mutant Type 1 and the Tm value of Mutant Type 2 were both 57˚C.
The Tm values in the case of Mixed Type 1 were 50˚C and 56˚C; the Tm values in the case of Mixed Type 2 were 50˚C and 56˚C; the Tm values in the case of Mixed Type 3 were 48˚C and 56˚C; and the Tm values in the case of Mixed Type 4 were 49˚C and 57˚C.
Fig. 3A to Fig. 3H show graphs obtained by the Tm analysis for Wild Type 1 (Fig. 3A), Wild Type 2 (Fig. 3C), Mutant Type 1 (Fig. 3B), Mutant Type 2 (Fig. 3D), Mixed Type 1 (Fig. 3E), Mixed Type 2 (Fig. 3F), Mixed Type 3 (Fig. 3G) and Mixed Type 4 (Fig. 3H), respectively. The vertical axis represents the temperature differentiation value of the fluorescence intensity, and the horizontal axis represents the temperature.

[Comparative Example 1]

Tm analysis was carried out using a fully-automated SNP analyzer (trade name: I-DENSY (trademark), manufactured by ARKRAY, Inc.) and the test reagents of the formulation shown in Table 13 below.
Table 13

| (Reaction Solution Volume: 50 μ l) | |
|---|---|
| 1 × Gene Taq Universal buffer ※<br>Probe: 3PB-CYP3A4*1 B-R3TK<br>Template (Complementary Strand) | <br>0.2 μ M<br>0.2 μ M |
| ※ manufactured by NIPPON GENE CO., LTD. | |

Details of the probe (SEQ ID NO:35) used in Table 13 above are shown in Table 14 below, and details of the templates (complementary strands) (ID-17 to ID-32) are shown in Table 15 below. The type of fluorescent dye is indicated in the parentheses at the 3' end of the probe.
Table 14

| Name | Sequence | mer | m(WT) | Tm(mt) | ⊿ | GC(%) |
|---|---|---|---|---|---|---|
| | taaatcgccTctctcCtgccc-(Pacific Blue) | 21 | 48.9 | 57.6 | 8.7 | 57.1 |
| Underlined capital letter: *1B (target SNP) Capital letter without underlining: a mutation (non-target SNP) other than *1B | | | | | | |

Table 15

| | | Target SNP |
|---|---|---|
| ID—17 | CYP3A4*1 B-AA-40-F | A/A |
| ID—18 | CYP3A4*1 B-GA-40-F | G/G |
| ID—19 | CYP3A4*1 B-AG-40-F | A/A |
| ID—20 | CYP3A4*1 B-GG-40-F | G/G |
| ID—21 | CYP3A4*1 B-AA-40-F | A/G |
| | CYP3A4*1B-GA-40-F | |
| ID—22 | CYP3A4*1 B-AA-40-F | A/A |
| | CYP3A4*1 B-AG-40-F | |
| ID—23 | CYP3A4*1 B-AA-40-F | A/G |
| | CYP3A4*1B-GG-40-F | |
| ID—24 | CYP3A4*1B-GA-40-F | A/G |
| | CYP3A4*1 B-AA-40-F | |
| ID—25 | CYP3A4*1B-GA-40-F | A/G |
| | CYP3A4*1 B-AG-40-F | |
| ID—26 | CYP3A4*1B-GA-40-F | G/G |
| | CYP3A4*1B-GG-40-F | |
| ID—27 | CYP3A4*1B-AG-40-F | A/A |
| | CYP3A4*1B-AA-40-F | |
| ID—28 | CYP3A4*1B-AG-40-F | A/G |
| | CYP3A4*1B-GA-40-F | |
| ID—29 | CYP3A4*1 B-AG-40-F | A/G |
| | CYP3A4*1B-GG-40-F | |
| ID—30 | CYP3A4*1B-GG-40-F | A/G |
| | CYP3A4*1 B-AA-40-F | |
| ID—31 | CYP3A4*1B-GG-40-F | G/G |
| | CYP3A4*1B-GA-40-F | |
| ID—32 | CYP3A4*1B-GG-40-F | A/G |
| | CYP3A4*1 B-AG-40-F | |

For the Tm analysis, treatment at 95˚C for 1 second and treatment at 40˚C for 60 seconds was sequentially carried out, and then the temperature was increased from 40˚C to 80˚C at a temperature increasing rate of 1˚C per 3 seconds, during which the change in fluorescence intensity was measured over time.

As a result of the Tm analysis, Fig. 4A to Fig. 4P, each showing the change in the fluorescence from the probe, were obtained. In the figures, the vertical axis represents the change in the fluorescence intensity per unit time (d(increase in fluorescence intensity)/t), and the horizontal axis represents the temperature (˚C). Fig. 4A shows the change in the fluorescence observed when ID-17 was used as the temperate; Fig. 4B shows the change in the fluorescence observed when ID-18 was used as the template; Fig. 4C shows the change in the fluorescence observed when ID-19 was used as the template; Fig. 4D shows the change in the fluorescence observed when ID-20 was used as the template; Fig. 4E shows the change in the fluorescence observed when ID-21 was used as the template; Fig. 4F shows the change in the fluorescence observed when ID-22 was used as the template; Fig. 4G shows the change in the fluorescence observed when ID-23 was used as the template; Fig. 4H shows the change in the fluorescence observed when ID-24 was used as the template; Fig. 4I shows the change in the fluorescence observed when ID-25 was used as the template; Fig. 4J shows the change in the fluorescence observed when ID-26 was used as the template; Fig. 4K shows the change in the fluorescence observed when ID-27 was used as the template; Fig. 4L shows the change in the fluorescence observed when ID-28 was used as the template; Fig. 4M shows the change in the fluorescence observed when ID-29 was used as the template; Fig. 4N shows the change in the fluorescence observed when ID-30 was used as the template; Fig. 4O shows the change in the fluorescence observed when ID-31 was used as the template; and Fig. 4P shows the change in the fluorescence observed when ID-32 was used as the template.

From these results, it was clarified that, when the probe employed in Comparative Example 1 is used, it is difficult to detect the presence or absence of a gene mutation at the 501st base, which is the target of detection, in the base sequence represented by SEQ ID NO:1, because the detection is affected by whether or not a non-target gene mutation occurs.

Thus, it was demonstrated that, according to the present invention, only a particular gene mutation to be detected can be detected from multiple gene mutations contained in a gene-encoding base sequence without being affected by the type of base present at a non-target gene mutation site.

The disclosures of Japanese Patent Application No. 2011-102987, filed May 2, 2011, Japanese Patent Application No. 2011-218114, filed September 30, 2011, and Japanese Patent Application No. 2012-082391, filed March 30, 2012, are incorporated by reference herein in their entirety.

All documents, patent applications and technical standards mentioned in the present specification are incorporated herein by reference to the same extent as if each individual document, patent application or technical standard is specifically and individually indicated to be incorporated by reference.

## Claims

**1.** A gene mutation detection probe for detection of a target gene mutation in a base sequence encoding a gene of interest that includes the target gene mutation and a non-target gene mutation, the probe comprising at least one oligonucleotide selected from the group consisting of oligonucleotides P1, P1-1, P1', and P1'-1:

the P1 oligonucleotide being an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and has an identity of at least 80% with a base sequence complementary to a part or all of the base sequence encoding the gene of interest;
the P1-1 oligonucleotide being an oligonucleotide that has, at the base position corresponding to the target gene mutation, a base complementary to either the wild-type base or a mutant base of the target gene mutation, and has, at the base position corresponding to the non-target gene mutation, a base complementary to neither the wild-type base nor a mutant base of the non-target gene mutation, and hybridizes with an oligonucleotide having the same base sequence as the base sequence encoding the gene of interest under stringent conditions;
the P1' oligonucleotide being an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and has an identity of at least 80% with a part or all of the base sequence encoding the gene of interest; and
the P1'-1 oligonucleotide being an oligonucleotide that has, at the base position corresponding to the target gene mutation, the same base as either the wild-type base or a mutant base of the target gene mutation, and has, at the base position corresponding to the non-target gene mutation, a base different from both the wild-type base and a mutant base of the non-target gene mutation, and hybridizes with an oligonucleotide having a base sequence complementary to the base sequence encoding the gene of interest under stringent conditions.

**2.** The gene mutation detection probe of claim 1, which satisfies any one of the following conditions (a) to (f):

(a) when the wild-type base at the base position of the non-target gene mutation is one of A or T, and the mutant base of the non-target gene mutation is the other one of A or T, the base complementary to neither the wild-type base nor the mutant base of the non-target gene mutation or the base different from both the wild-type base and the mutant base of the non-target gene mutation is C or G;
(b) when the wild-type base at the base position of the non-target gene mutation is one of C or G, and the mutant base of the non-target gene mutation is the other one of C or G, the base complementary to neither the wild-type base nor the mutant base of the non-target gene mutation or the base different from both the wild-type base and the mutant base of the non-target gene mutation is A or T;
(c) when the wild-type base at the base position of the non-target gene mutation is one of A or C, and the mutant base of the non-target gene mutation is the other one of A or C, the base complementary to neither the wild-type base nor the mutant base of the non-target gene mutation is A or C, and the base different from both the wild-type base and the mutant base of the non-target gene mutation is T or G;
(d) when the wild-type base at the base position of the non-target gene mutation is one of A or G, and the mutant base of the non-target gene mutation is the other one of A or G, the base complementary to neither the wild-type base nor the mutant base of the non-target gene mutation is A or G, and the base different from both the wild-type base and the mutant base of the non-target gene mutation is T or C;
(e) when the wild-type base at the base position of the non-target gene mutation is one of T or C, and the mutant base of the non-target gene mutation is the other one of T or C, the base complementary to neither the wild-type base nor the mutant base of the non-target gene mutation is T or C, and the base different from both the wild-type base and the mutant base of the non-target gene mutation is A or G; or
(f) when the wild-type base at the base position of the non-target gene mutation is one of T or G, and the mutant base of the non-target gene mutation is the other one of T or G, the base complementary to neither the wild-type base nor the mutant base of the non-target gene mutation is T or G, and the base different from both the wild-type base and the mutant base of the non-target gene mutation is A or C.

**3.** The gene mutation detection probe of claim 1 or 2, wherein the oligonucleotide is a fluorescently-labeled oligonucleotide.

**4.** The gene mutation detection probe of claim 3, wherein the base at the 3' or 5' end of the fluorescently-labeled oligonucleotide is a cytosine labeled with a fluorescent dye.

**5.** The gene mutation detection probe of claim 3 or 4, wherein the fluorescently-labeled oligonucleotide is fluorescently-labeled at any of the first to the third bases from the 3' or 5' end.

**6.** The gene mutation detection probe of any one of claims 1 to 5, which is a probe for melting curve analysis.

**7.** The gene mutation detection probe of any one of claims 3 to 6, wherein the fluorescence intensity when the fluorescently-labeled oligonucleotide is hybridized with a target sequence thereof is decreased or increased as compared to the fluorescence intensity when the fluorescently-labeled oligonucleotide is not hybridized with the target sequence.

**8.** The gene mutation detection probe of any one of claims 3 to 7, wherein the fluorescence intensity when the fluorescently-labeled oligonucleotide is hybridized with a target sequence thereof is decreased as compared to the fluorescence intensity when the fluorescently-labeled oligonucleotide is not hybridized with the target sequence.

**9.** A method of detecting a gene mutation in a sample containing nucleic acid which may contain a target gene mutation, comprising contacting the gene mutation detection probe of any one of claims 1 to 8 with said sample and detecting the presence or absence of said target gene mutation.

**10.** The method of detecting a gene mutation according to claim 9, wherein the method comprises:

(I) contacting the gene mutation detection probe of any one of claims 3 to 8 with a single-stranded nucleic acid in said sample, to obtain a hybrid;
(II) dissociating the hybrid by changing the temperature of the sample containing the hybrid, and measuring the change in fluorescence signal due to the dissociation of the hybrid;
(III) obtaining the Tm value, which is the dissociation temperature of the hybrid, based on the change in fluorescence signal; and
(IV) detecting the presence or absence of the target gene mutation on the single-stranded nucleic acid in the sample based on the Tm value.

**11.** The method of detecting a target gene mutation of claim 10, further comprising amplifying the nucleic acid before or simultaneously with obtaining the hybrid in (I).

**12.** A gene mutation detection reagent kit that comprises the gene mutation detection probe of any one of claims 1 to 8.

**13.** The gene mutation detection reagent kit of claim 12, further comprising primers capable of amplifying a base sequence having a region to which the gene mutation detection probe of any one of claims 1 to 8 hybridizes.

FIG. 1A

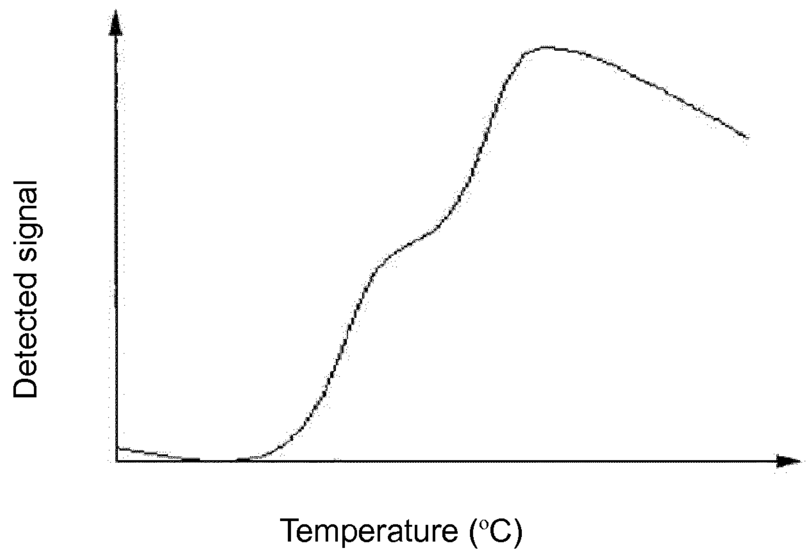
Detected signal
Temperature (°C)

FIG. 1B

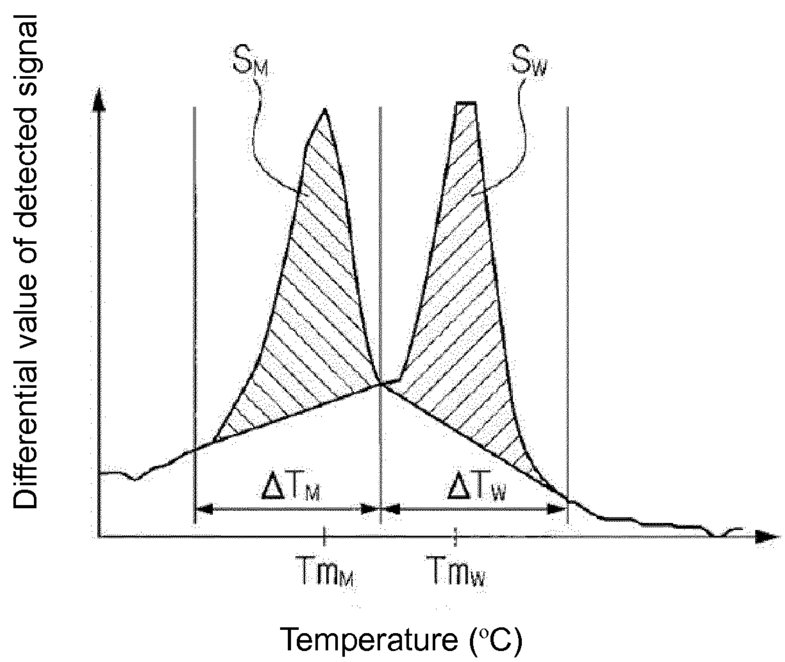
Differential value of detected signal
SM
SW
ΔTM
ΔTW
TmM
TmW
Temperature (°C)

Fig.2A

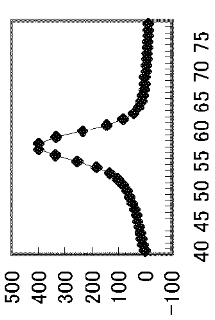


Fig.2B

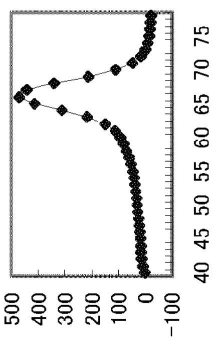


Fig.2C

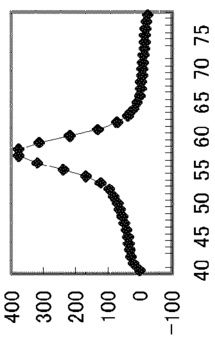


Fig.2D

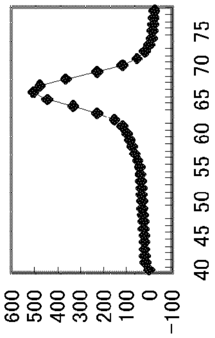


Fig.2E

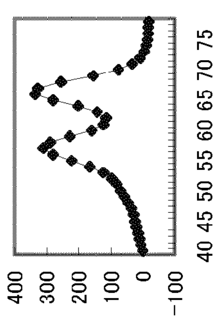


Fig.2F

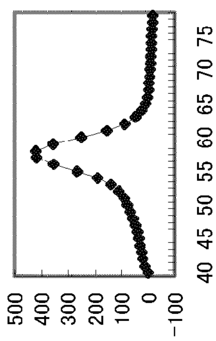


Fig.2G

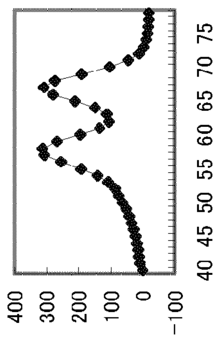


Fig.2H

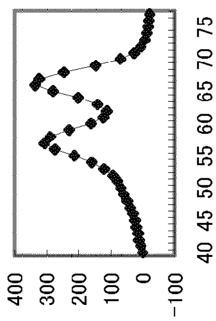

Fig.2I

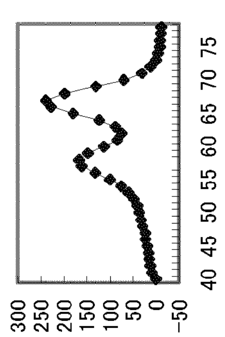


Fig.2J

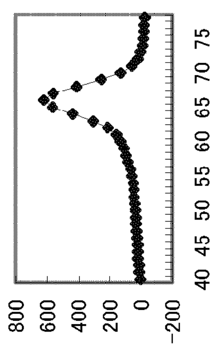


Fig.2K

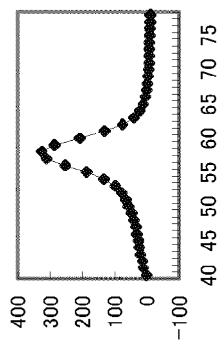


Fig.2L

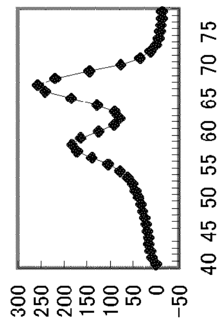


Fig.2M

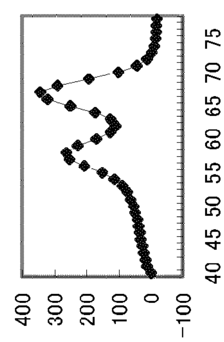


Fig.2N

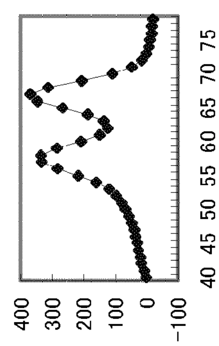


Fig.2O

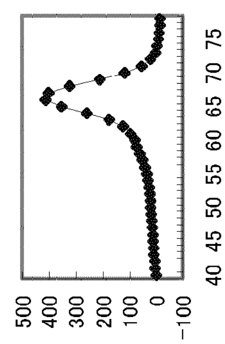


Fig.2P

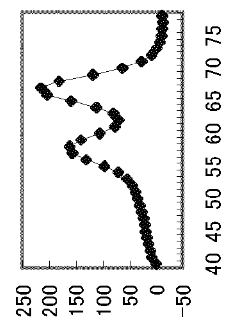

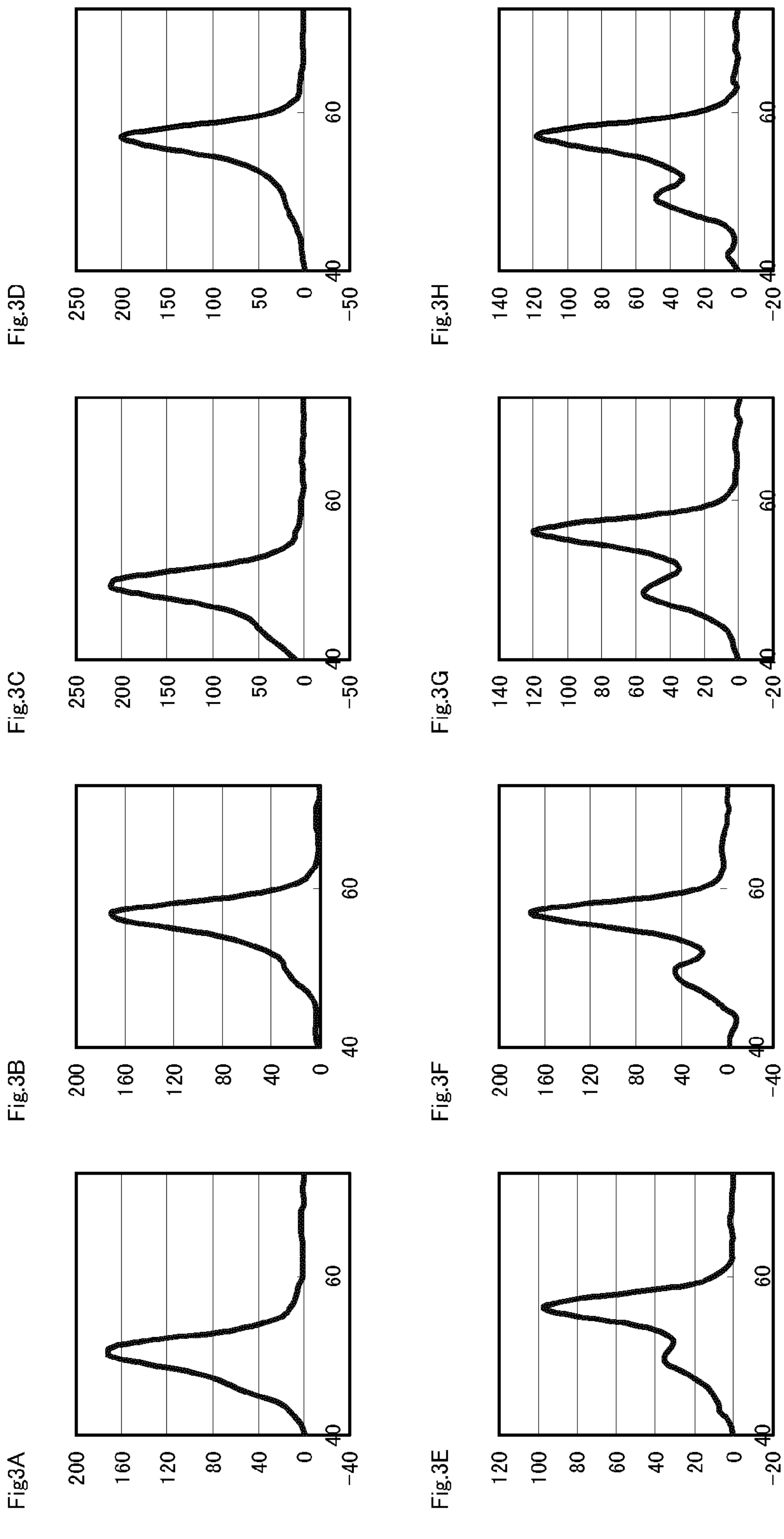
Fig3A
Fig.3B
Fig.3C
Fig.3D
Fig.3E
Fig.3F
Fig.3G
Fig.3H

Fig.4A

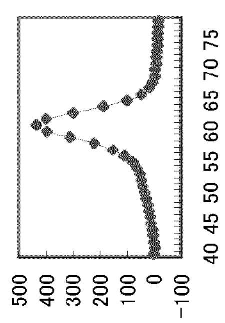


Fig.4B

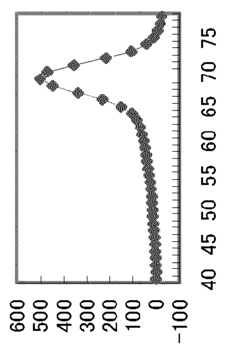


Fig.4C

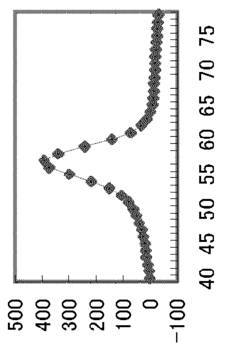


Fig.4D

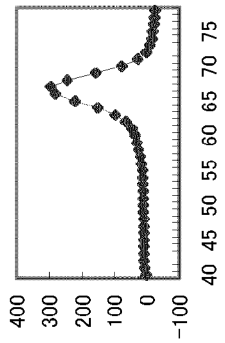


Fig.4E

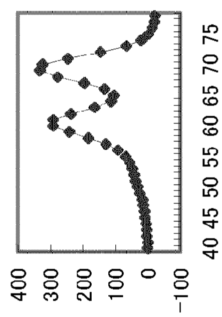


Fig.4F

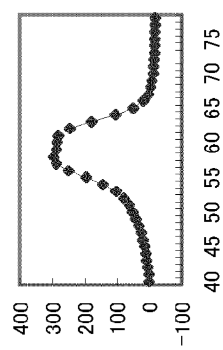


Fig.4G

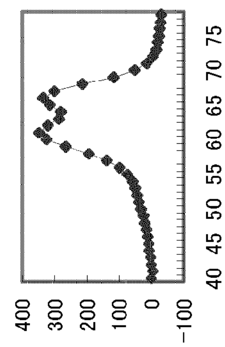


Fig.4H

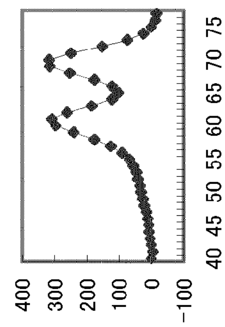

Fig.4I

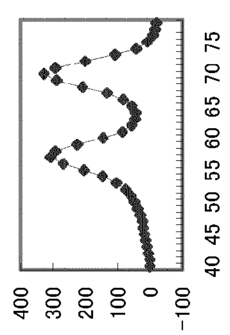


Fig.4J

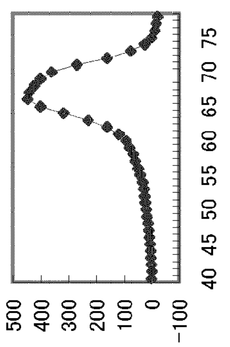


Fig.4K

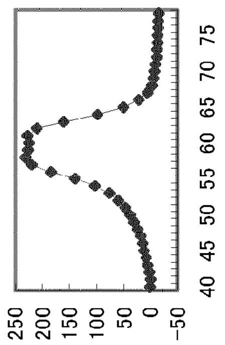


Fig.4L

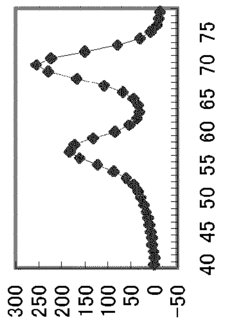


Fig.4M

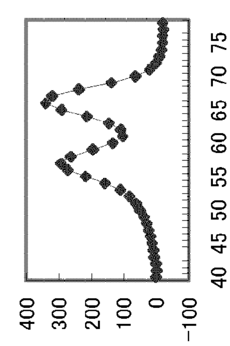


Fig.4N

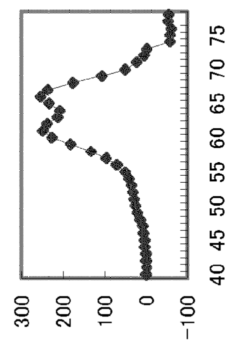


Fig.4O

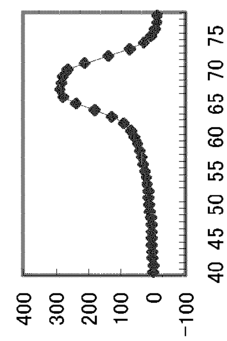


Fig.4P

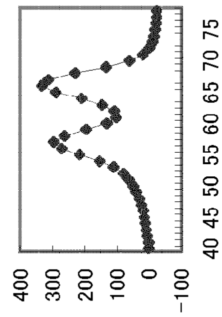

Europäisches Patentamt
European Patent Office
Office européen des brevets

# EUROPEAN SEARCH REPORT

Application Number
EP 12 16 6067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2005/035545 A2 (EPOCH BIOSCIENCES INC [US]; BELOUSOV YEVGENIY S [US]; DEMPCY ROBERT O) 21 April 2005 (2005-04-21)<br>* abstract *<br>* page 8, line 28 - page 9, line 2; examples 2-6 *<br>* page 14 *<br>* page 45 - page 48 *<br>* page 52, paragraph 3 *<br>* page 54, paragraph 3; claims 15-42; figure 1 *<br>----- | 1,3-13<br><br><br>2 | INV.<br>C12Q1/68 |
| Y<br><br><br><br><br><br><br><br><br><br><br><br>A | BURGNER DAVID ET AL: "Improved allelic differentiation using sequence-specific oligonucleotide hybridization incorporating an additional base-analogue mismatch",<br>NUCLEOSIDES, NUCLEOTIDES AND NUCLEIC ACIDS, TAYLOR & FRANCIS, PHILADELPHIA, PA, vol. 23, no. 5, 1 May 2004 (2004-05-01), pages 755-765, XP009099652, ISSN: 1525-7770, DOI: 10.1081/NCN-120039216<br>* abstract *<br>* page 758; figure 1 *<br>* page 762, paragraph 2 - page 763, paragraph 1 *<br>----- | 2<br><br><br><br><br><br><br><br><br><br><br><br>1,3-13 | TECHNICAL FIELDS SEARCHED (IPC)<br>C12Q |
| A | GUO Z ET AL: "ENHANCED DISCRIMINATION OF SINGLE NUCLEOTIDE POLYMORPHISMS BY ARTIFICIAL MISMATCH HYBRIDIZATION",<br>NATURE BIOTECHNOLOGY, NATURE PUBLISHING GROUP, NEW YORK, NY, US, vol. 15, 1 April 1997 (1997-04-01), pages 331-335, XP000867755, ISSN: 1087-0156, DOI: 10.1038/NBT0497-331<br>* the whole document *<br>-----<br>-/-- | 1-13 | |

The present search report has been drawn up for all claims

| Place of search | Date of completion of the search | Examiner |
|---|---|---|
| Munich | 18 July 2012 | Tilkorn, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 6067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/095941 A1 (SEEGENE INC [KR]; CHUN JONG-YOON [KR]) 14 September 2006 (2006-09-14) * page 15, line 19 - page 16, line 14; examples 2,3,8 * ----- | 1-13 | |
| A | US 6 027 880 A (CRONIN MAUREEN T [US] ET AL) 22 February 2000 (2000-02-22) * column 21, line 45 - line 55 * ----- | 1-13 | |
| | | | TECHNICAL FIELDS SEARCHED (IPC) |

The present search report has been drawn up for all claims

| Place of search | Date of completion of the search | Examiner |
|---|---|---|
| Munich | 18 July 2012 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 12 16 6067

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2012

| Patent document cited in search report | Publication date | Patent family member(s) | Publication date |
|---|---|---|---|
| WO 2005035545 A2 | 21-04-2005 | AU 2004279810 A1 | 21-04-2005 |
| | | CA 2540551 A1 | 21-04-2005 |
| | | EP 1670928 A2 | 21-06-2006 |
| | | JP 2007510401 A | 26-04-2007 |
| | | US 2005118623 A1 | 02-06-2005 |
| | | US 2009087922 A1 | 02-04-2009 |
| | | WO 2005035545 A2 | 21-04-2005 |
| WO 2006095941 A1 | 14-09-2006 | AT 529534 T | 15-11-2011 |
| | | BR PI0609031 A2 | 16-11-2010 |
| | | CN 101189336 A | 28-05-2008 |
| | | DK 1856257 T3 | 27-02-2012 |
| | | EP 2365078 A1 | 14-09-2011 |
| | | EP 2365079 A1 | 14-09-2011 |
| | | ES 2375571 T3 | 02-03-2012 |
| | | JP 4903725 B2 | 28-03-2012 |
| | | JP 2008531040 A | 14-08-2008 |
| | | JP 2012065655 A | 05-04-2012 |
| | | KR 20070111543 A | 21-11-2007 |
| | | KR 20100099333 A | 10-09-2010 |
| | | NZ 561166 A | 30-09-2011 |
| | | PT 1856257 E | 02-01-2012 |
| | | US 2008305478 A1 | 11-12-2008 |
| | | US 2012135473 A1 | 31-05-2012 |
| | | WO 2006095941 A1 | 14-09-2006 |
| | | ZA 200707514 A | 31-12-2008 |
| US 6027880 A | 22-02-2000 | US 6027880 A | 22-02-2000 |
| | | US 2003165823 A1 | 04-09-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002119291 A
- JP 2011102987 A
- JP 2011218114 A
- JP 2012082391 A


### Non-patent literature cited in the description


- **J. SAMBROOK E.** Molecular Cloning. Cold Spring Harbor Lab. Press, 2001
- **J. SAMBROOK et al.** Molecular Cloning. Cold Spring Harbor Lab. Press, 2001
- *Clin. Pharmacol. Ther.,* 2006, vol. 80, 179-191